Europäisches Patentamt

European Patent Office ·

Office européen des brevets

(19)

(11) Publication number: **0 323 722**
**A1**

## EUROPEAN PATENT APPLICATION

(21) Application number: **88311924.0**

(22) Date of filing: **16.12.88**

(51) Int. Cl.⁴: **C12N 15/00 , C12N 9/64 , A61K 37/00**

(30) Priority: **18.12.87 US 134981**

(43) Date of publication of application:
**12.07.89 Bulletin 89/28**

(84) Designated Contracting States:
**AT BE CH DE ES FR GB GR IT LI LU NL SE**

(71) Applicant: **CHIRON CORPORATION**
**4560 Horton Street**
**Emeryville California 94608(US)**

(72) Inventor: **Valenzuela, Pablo**
**455 Upper Terrace No.3**
**San Francisco California 94117(US)**
Inventor: **Brake, Anthony**
**2115, Los Angeles Avenue**
**Berkeley California 94707(US)**
Inventor: **Randolph, Anne**
**1421 Grand Avenue**
**Piedmont California 94610(US)**

(74) Representative: **Goldin, Douglas Michael et al**
**J.A. KEMP & CO. 14, South Square Gray's Inn**
**London WC1R 5EU(GB)**

(54) Compositions and method for recombinant production of crotalidus venom fibrolase.

(57) Recombinant DNA encoding several forms of southern copperhead fibrolase, yeast expression vectors containing such DNA, and the production of southern copperhead fibrolase in yeast are disclosed.

EP 0 323 722 A1

# COMPOSITIONS AND METHODS FOR RECOMBINANT PRODUCTION OF CROTALIDUS VENOM FIBROLASE

## Technical Field

This invention is in the fields of enzyme chemistry, genetic engineering, and thrombolytic therapy. More particularly, it relates to the recombinant production of the fibrinolytic enzymes, present in the venom of snakes of the family Crotalidae, such as Agkistrodon contortrix contortrix (southern copperhead) fibrolase.

## Background Art

Thrombolytic therapy is an established procedure for treatment of various thromboembolic conditions such as pulmonary embolism, thrombophlebitis, and arterial thromboembolism. Many of the thrombolytic agents being investigated act indirectly by activating plasminogen throughout the circulation and are, therefore, not directed specifically towards the thrombus. These plasminogen-activating fibrinolytic agents have perceived disadvantages in therapy, such as inducing sufficient plasmin to deplete clotting factors to levels that enhance the probability of hemorrhagic complications after thrombolytic therapy. These perceived problems have led to the investigation of direct-acting thrombolytic agents, such as the fibrinolytic enzymes present in many snake venoms.

Numerous direct acting fibrinolytic enzymes have been identified in snake venoms. Among those isolated from Crotalidus venom are southern copperhead fibrolase, the purification and characterization of which is described in U.S. patent No. 4,610,879 and green pit viper fibrolase described in EPA publication no. 0020780. Preliminary investigation of southern copperhead fibrolase indicates it offers promise as a safe, effective, direct-acting agent for thrombolysis. Despite the therapeutic promise of this agent, however, it will be impractical to carry out extended clinical investigation of native fibrolase because of the scarcity of the source venom. Also fibrolase purified from venom pooled from a number of snakes is likely to be heterogenous due to allelic/polymorphic variation between individual snakes. A principal purpose of the present invention is to provide a more practical means for obtaining homogenous Crotalidus fibrolases than purifying them from venom.

## Disclosure of the Invention

Accordingly, one aspect of the invention is recombinant DNA encoding a Crotalidus fibrolase, such as southern copperhead fibrolase.

Cloning and expression vectors containing such recombinant DNA are another aspect of the invention.

Hosts such as transformed bacteria, yeast, and mammalian cells which contain such expression vectors and are capable of producing active recombinant Crotalidus fibrolase are another aspect of the invention.

Methods for producing Crotalidus fibrolase which employ such hosts are still another aspect of the invention.

Recombinant homogeneous Crotalidus fibrolase which is free of other Crotalidus proteins is a further aspect of the invention.

## Brief Description of the Drawings

Figure 1 shows the nucleotide sequence and the deduced amino acid sequence of a southern copperhead fibrolase clone designated SVG-48 (or FIB 48).

Figure 2 shows the partial nucleotide sequence and the deduced partial amino acid sequence of another southern copperhead fibrolase clone designated SVG-51 (or FIB 51).

Figure 3 shows a comparison of the nucleotide sequences of the coding strands of the fibrolases of Figures 1 and 2. Nucleotide variances between the sequences are asterisked.

Figure 4 shows a comparison of the amino acid sequences (using single letter amino acid designations) of the fibrolases of Figures 1 and 2. Amino acid variances between the two sequences are asterisked.

Figure 5 is a flow diagram depicting a scheme for preparing the yeast expression plasmids pAB24F248 or pAB24F251 which can be used to produce southern copperhead fibrolase in yeast.

Figure 6 shows the nucleotide sequence of the 1341 bp ADH2-GAPDH promoter fragment referred to in the Examples.

Figure 7 shows the nucleotide sequence and amino acid sequence of an α-factor profibrolase fusion construct with a Lys-Arg processing site at the pro-mature junction (Example 5, infra).

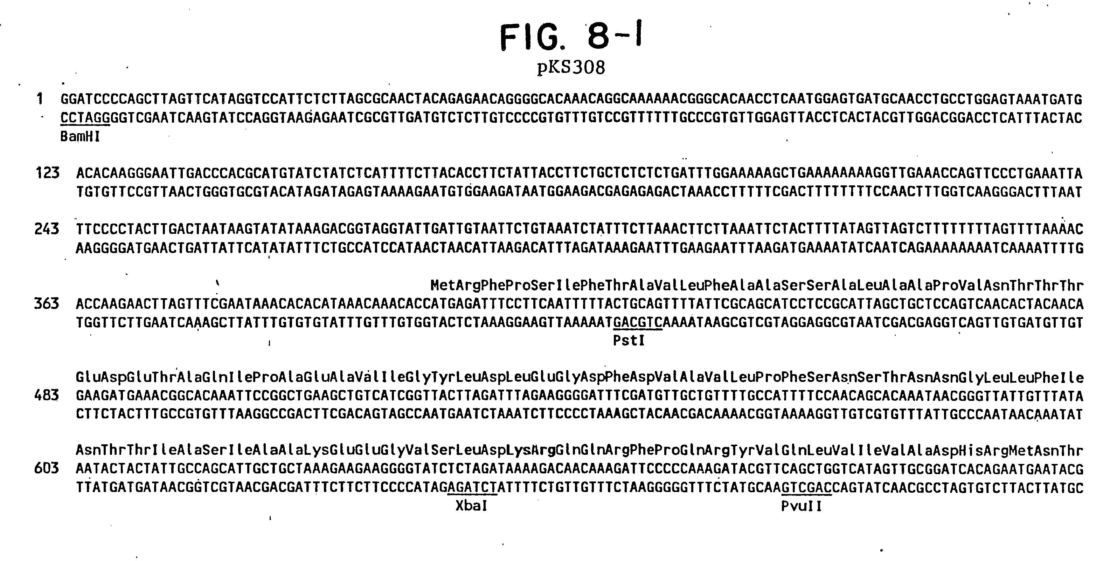

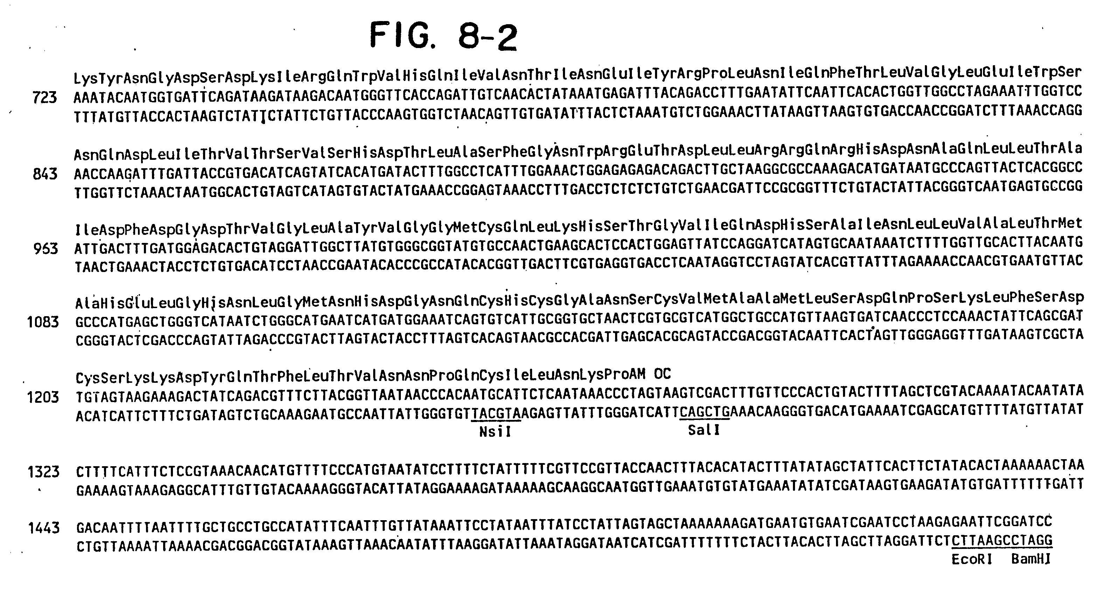

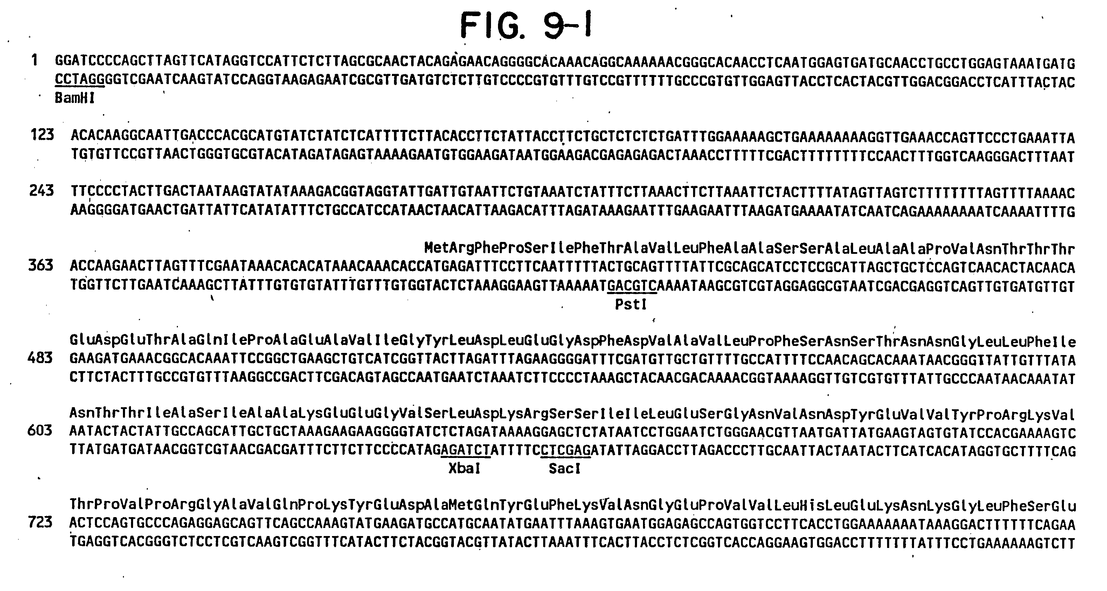

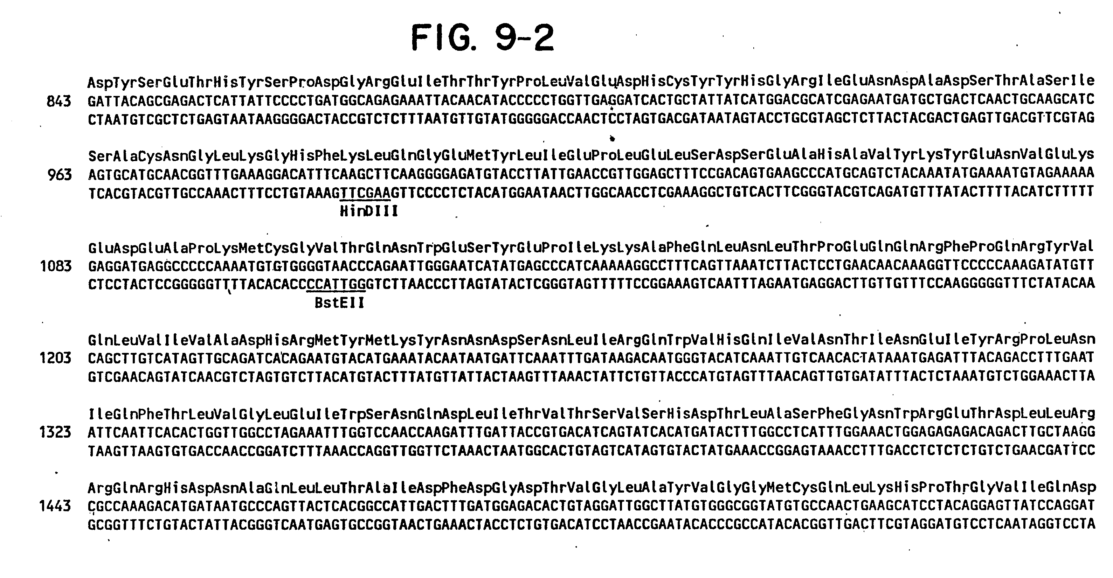

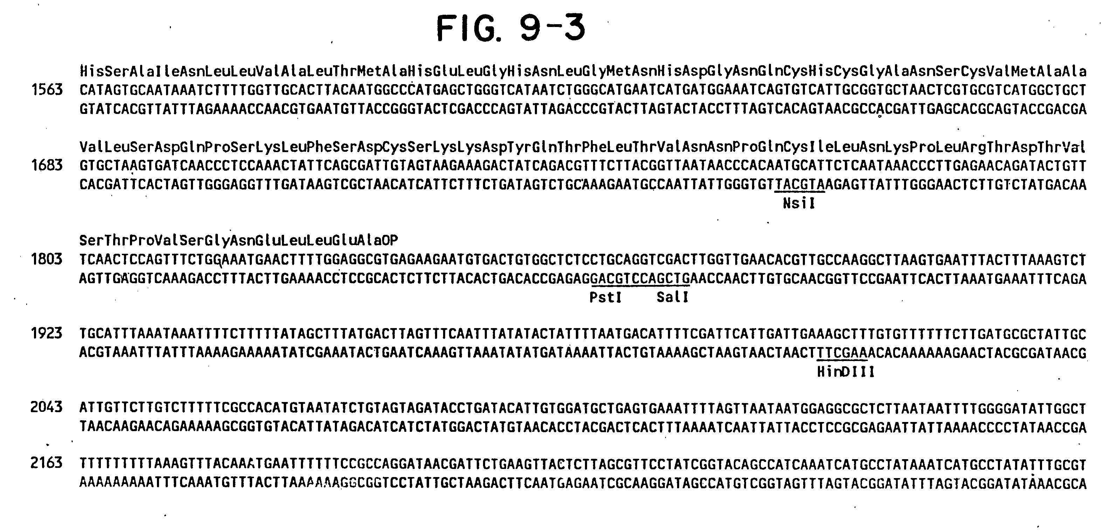

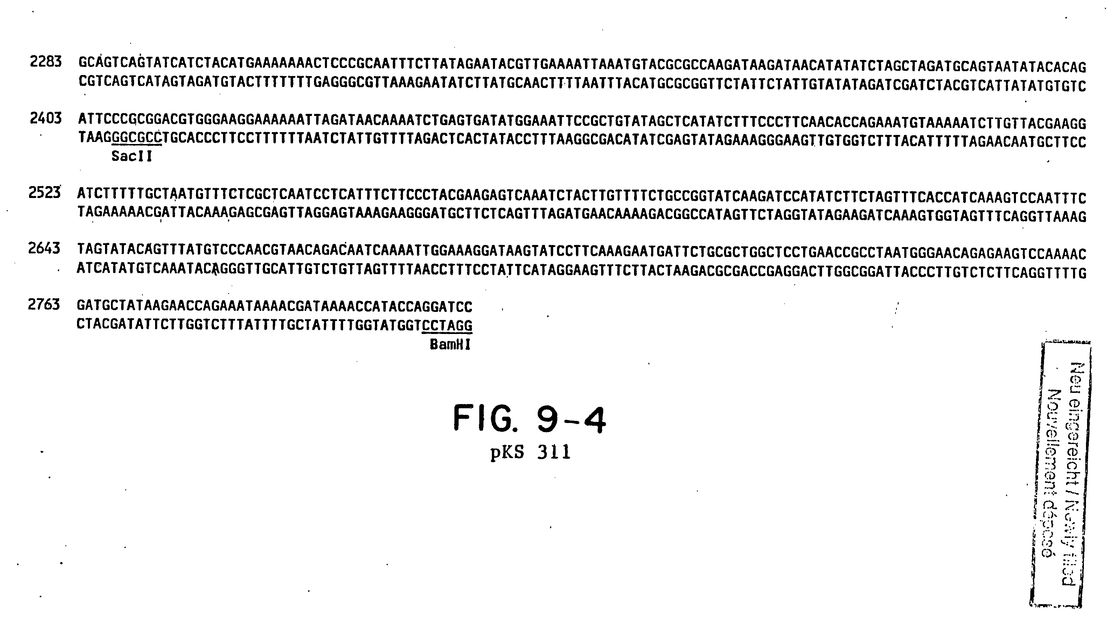

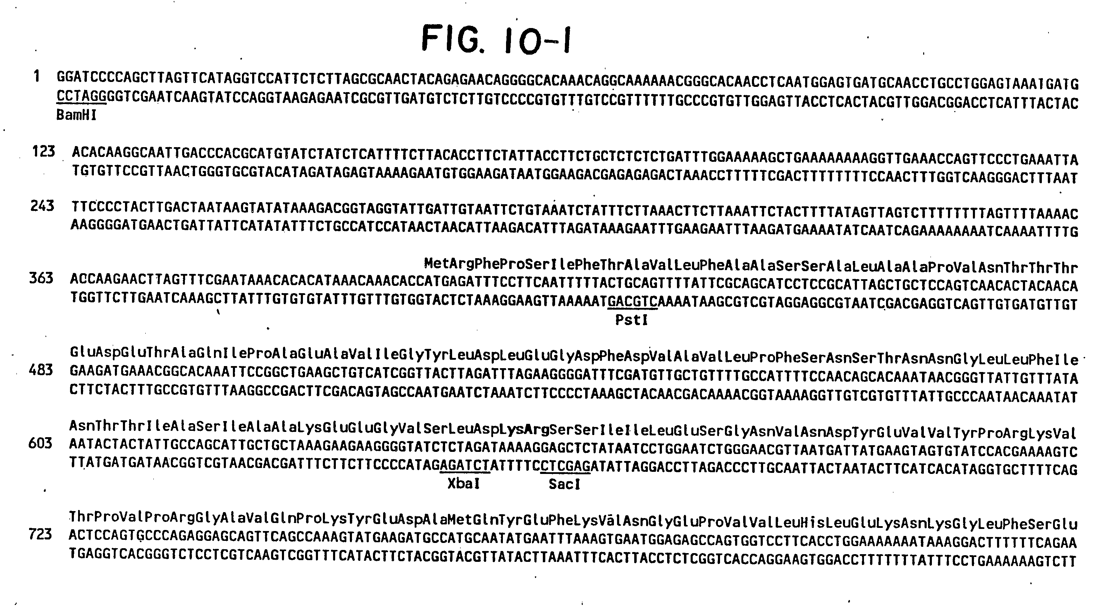

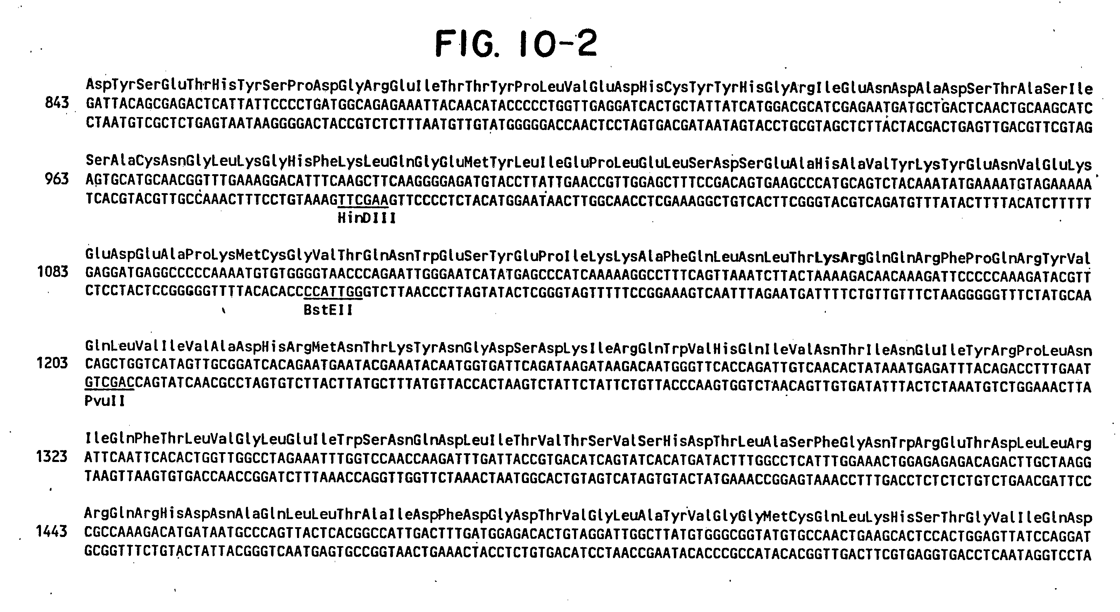

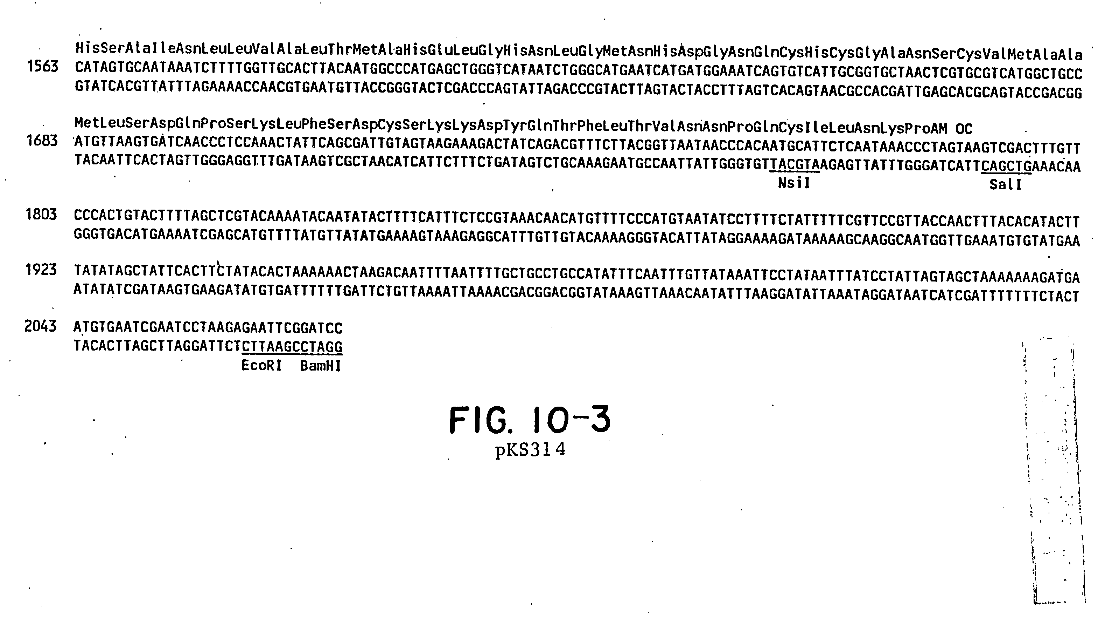

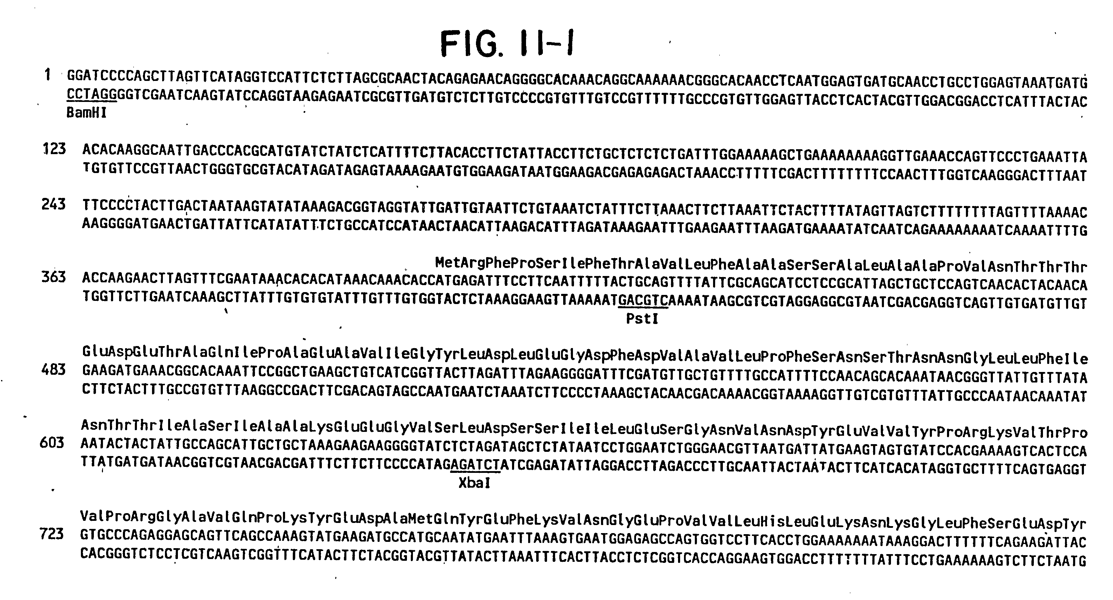

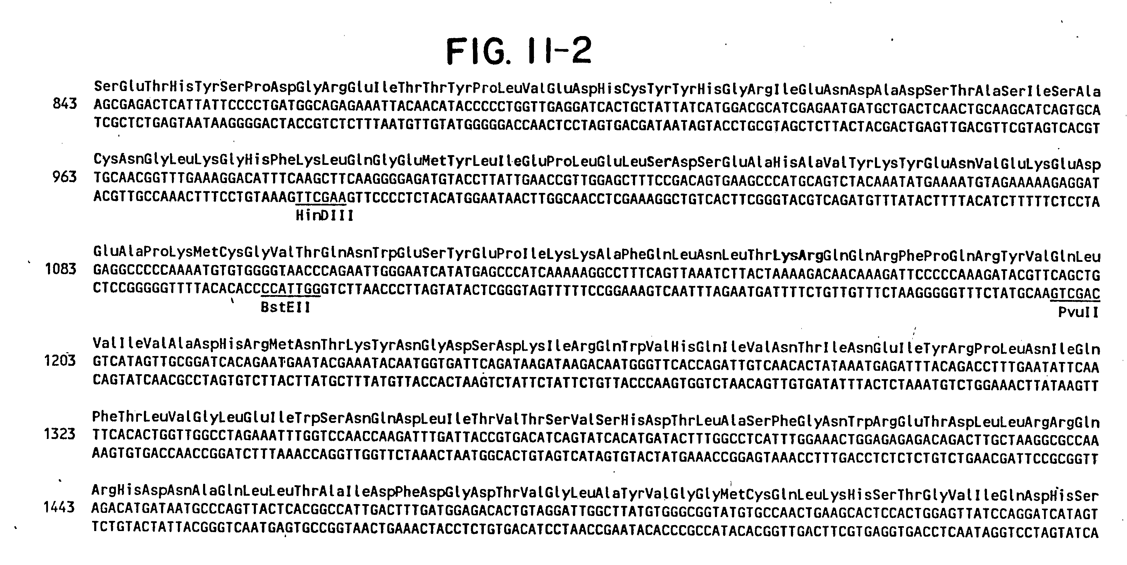

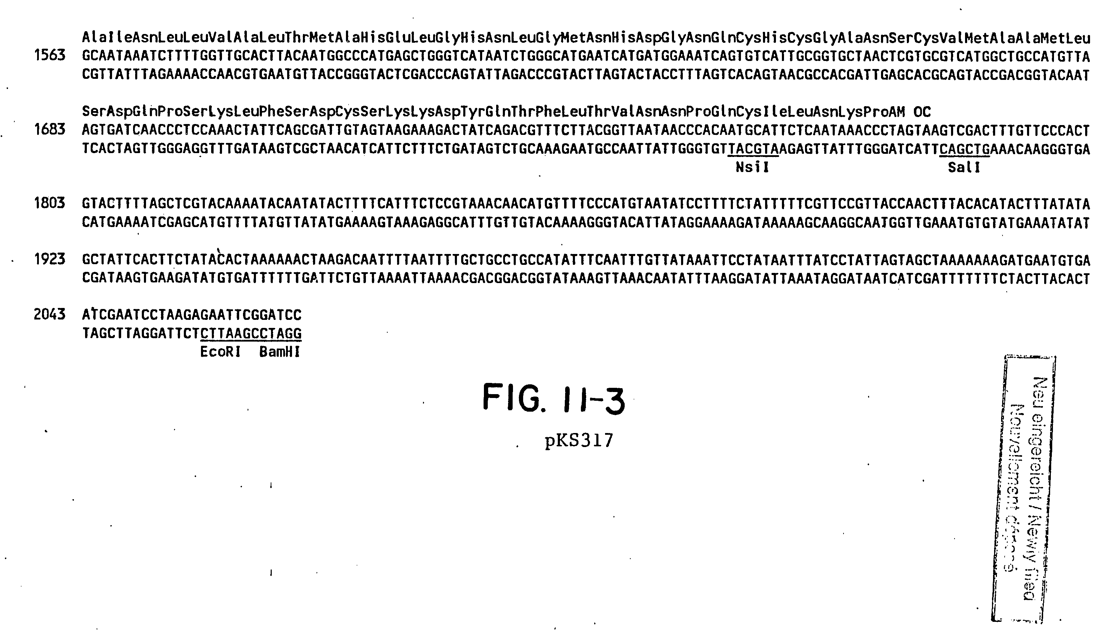

Figures 8, 9, 10 and 11 are the nucleotide and corresponding amino acid sequences of the α-factor profibrolase fusion constructs of plasmids pKS308, pKS311, pKS314 and pKS317 of Example 5, infra.

Modes for Carrying Out the Invention

1. Definitions

The term "recombinant" as used herein to characterize DNA encoding Crotalidus fibrolase intends DNA of genomic, cDNA, semisynthetic, or synthetic origin which, by virtue of its origin or manipulation is (1) not associated with all or a portion of the DNA with which it is associated in nature or in the form of a library and/or (2) linked to DNA other than that to which it is linked in nature. "Recombinant" as used to describe Crotalidus fibrolase intends protein produced from such DNA.

A "replicon" is any genetic element (e.g., a plasmid, a chromosome, a virus) that behaves as an autonomous unit of polynucleotide replication within a cell; i.e., capable of replication under its own control.

A "vector" is a replicon in which another polynucleotide segment is attached, so as to bring about the replication and/or expression of the attached segment. An "expression vector" refers to a vector capable of autonomous replication or integration and contains control sequences which direct the transcription and translation of the southern copperhead fibrolase DNA in an appropriate host.

A "coding sequence" is a polynucleotide sequence which is transcribed and/or translated into a polypeptide.

A "promoter sequence" is a DNA regulatory region capable of binding RNA polymerase and initiating transcription of a downstream (i.e., in the 3′ direction) coding sequence.

A coding sequence is "under the control" of the promoter sequence in a cell when transcription of the coding sequence results from the binding of RNA polymerase to the promoter sequence; translation of the resulting mRNA then results in the polypeptide encoded within the coding sequence.

"Operably linked" refers to a juxtaposition wherein the components are configured so as to perform their usual function. Thus, control sequences operably linked to a coding sequence are capable of effecting the expression of the coding sequence.

"Control sequences" refers to those sequences which control the transcription and/or translation of the coding sequence(s); these may include, but are not limited to, promoter sequences, transcriptional initiation and termination sequences, and translational initiation and termination sequences. In addition, "control sequences" refers to sequences which control the processing of the polypeptide encoded within the coding sequence; these may include, but are not limited to sequences controlling secretion, protease cleavage, and glycosylation of the polypeptide.

"Transformation" is the introduction of an exogenous polynucleotide into a host cell. The exogenous polynucleotide may be maintained as a plasmid, or alternatively, may be integrated within the host genome.

"Crotalidae" denotes the family of snakes commonly known as pit vipers. Members of the Crotalidae family include species of the subfamilies Agkistrodon (A), Crotalus (C), Bothrops (B) and Trimeresurus (T). Examples of snake species of this family are A. acutus, A. bilineatus, A. caliginosus, A. contortrix, A. halys, A. halys blomhoffii, A. hypnale, A. mokasen, A. piscivorous, A. rhodostoma, A. saxatilis, B. alternatus, B. atrox, B. bilineatus, B. caribbaeus, B. godmani, B. itapetiningae, B. jararaca, B. jararacussu, B. lanceolatus, B. lansbergii, B. nasuta, B. neuwiedi, B. nigroviridis marchi, B. nummifer (B. nummifera), B. schlegelii, C. adamanteus, C. atrox, C. basiliscus, C. cerastes, C. confluentus, C. durissus, C. durissus terrificus, C. horridus, C. lepidus, C. mitchellii, C. molossus, C. ruber, C. scutulatus, C. tigris, C. unicolor, C. viridis lutosus, C. viridis oreganus, L. mutus, S. catenatus, S. miliarius, S. ravus, T. albolabris, T. elegans, T. flavoviridis, T. gramineus, T. monticola, T. mucrosquamatus, T. okinavensis, T. popeorum, T. purpureomaculatus, T. stejnegeri, T. tokarensis, T. wagleri.

2. Recombinant Southern Copperhead DNA

3

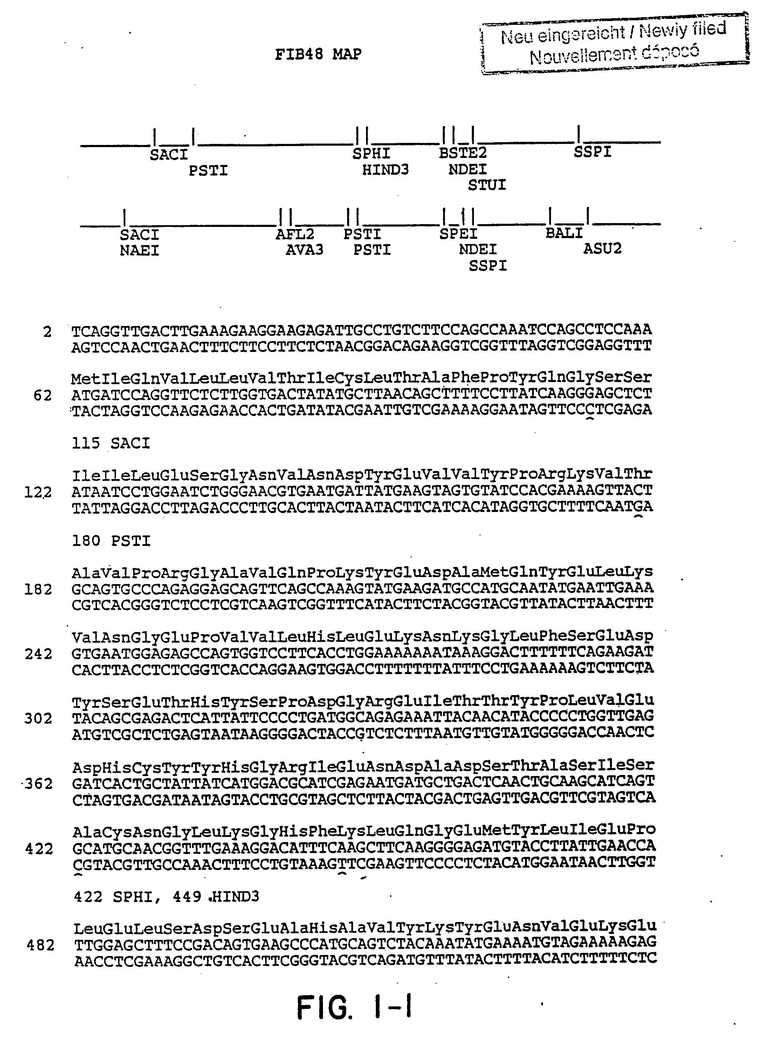

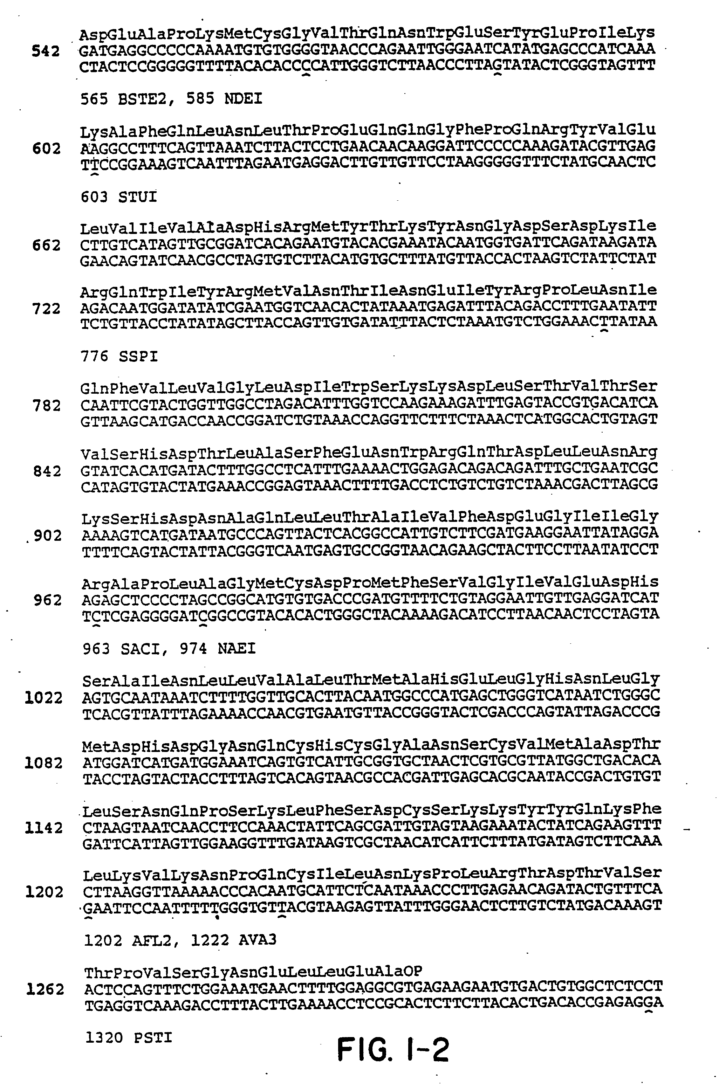

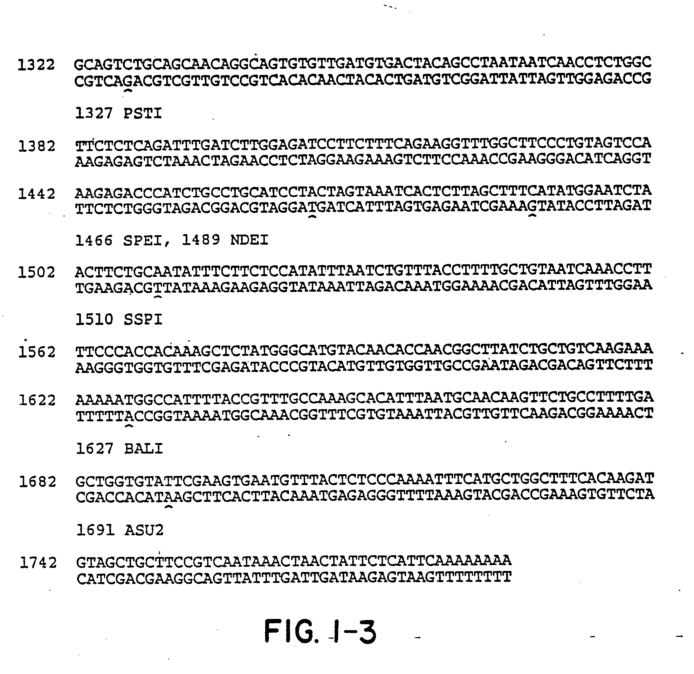

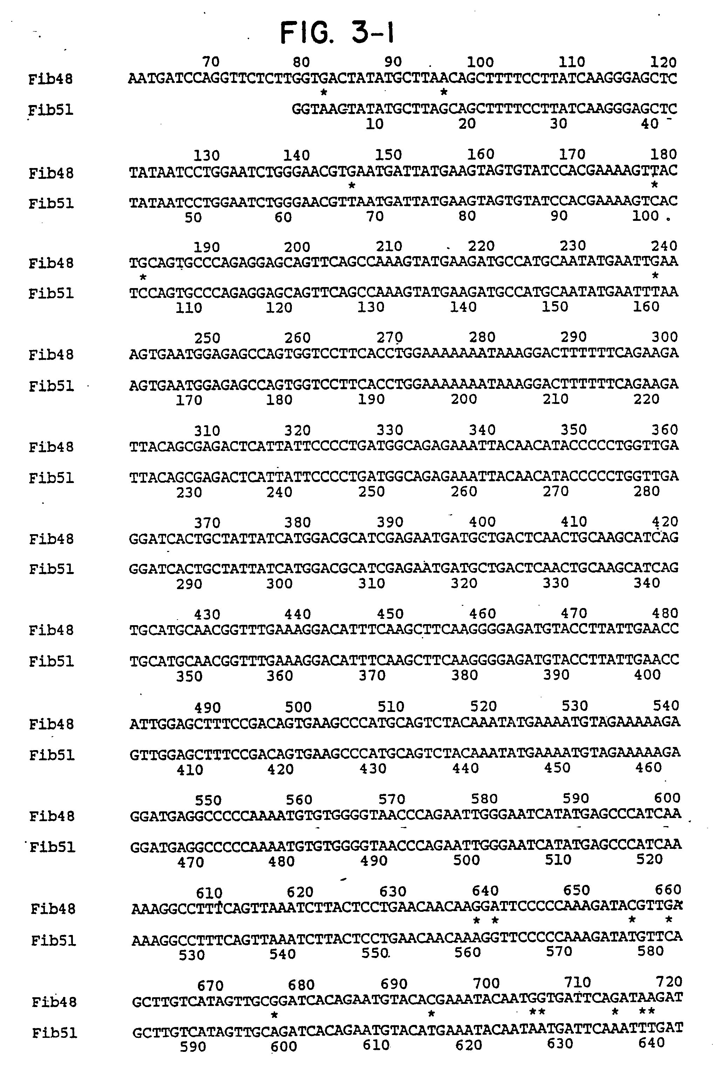

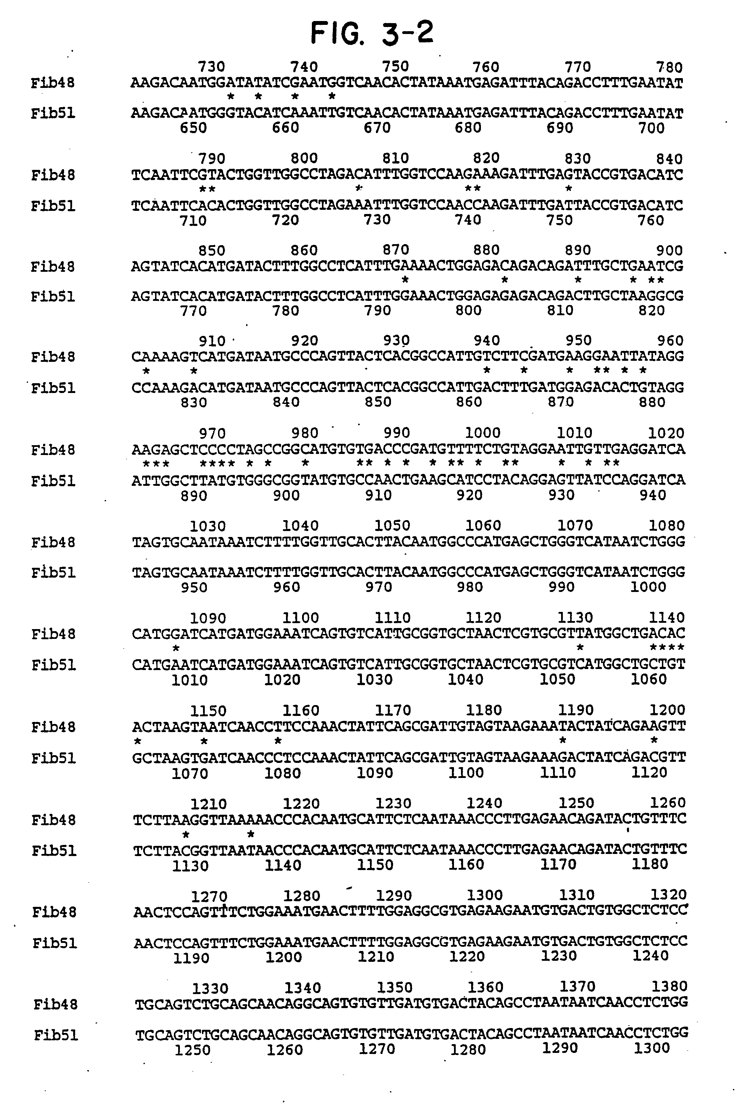

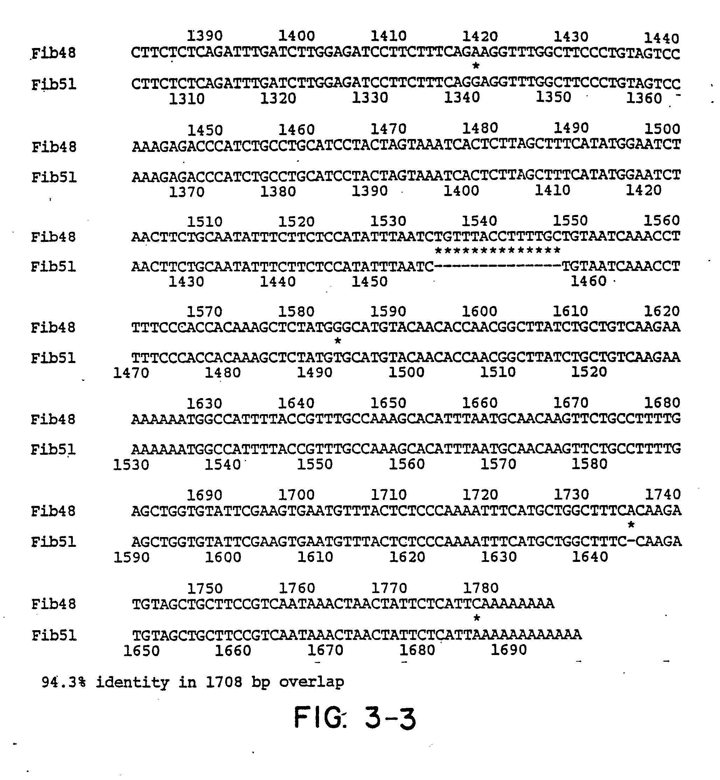

Figures 1-4 show the nucleotide sequences and deduced amino acid sequences of two southern copperhead fibrolase clones, designated pSVG-48 (or Fib 48) and pSVG-51 (or Fib 51). Fib 48 includes the complete sequence for southern copperhead preprofibrolase whereas Fib 51 lacks a short 5' sequence and initiator codon.

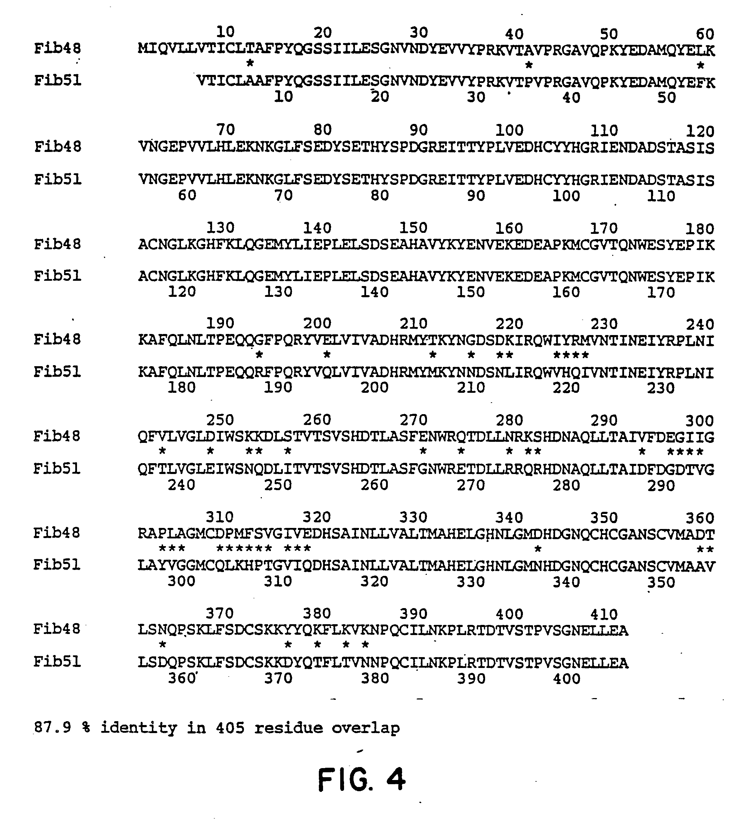

Based on the Fib 48 and Fib 51 sequences and amino acid sequences it is apparent that there are polymorphic variations and/or allelic variations of southern copperhead fibrolase. Amino acid sequencing of native protein shows different residues at amino acids 210 (Tyr-----Asn) and 360 (Thr/Val-----Met) (Figure 4) from the sequences predicted from the two clones thus providing further evidence of such variation.

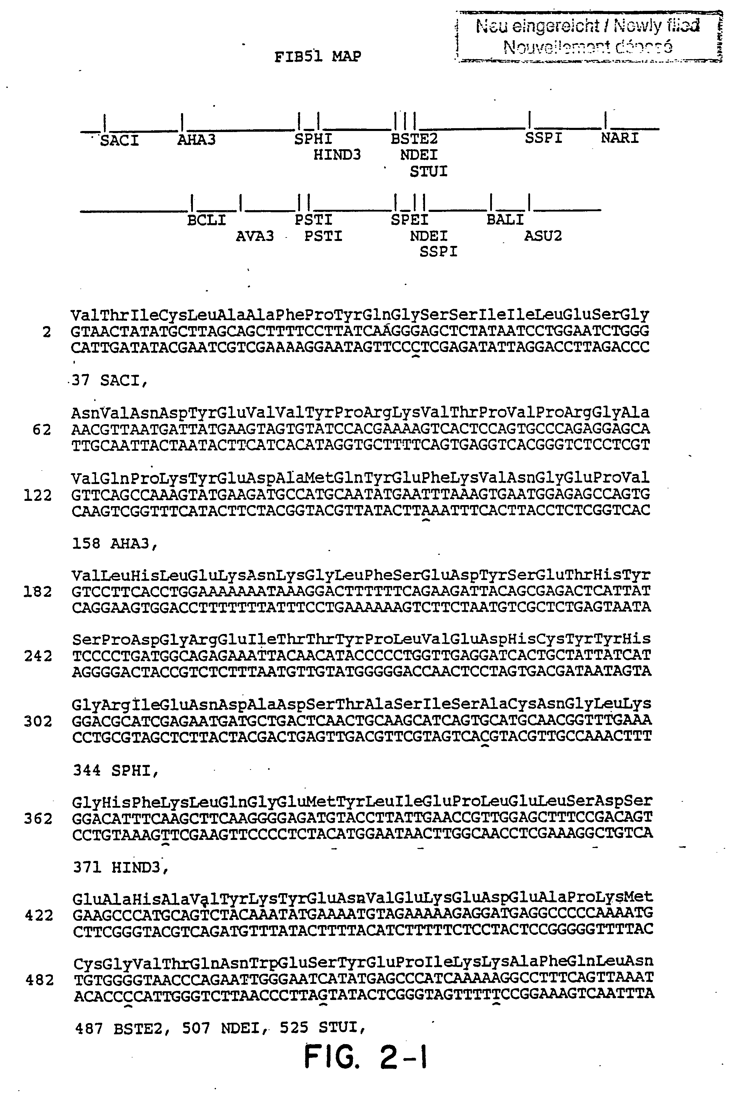

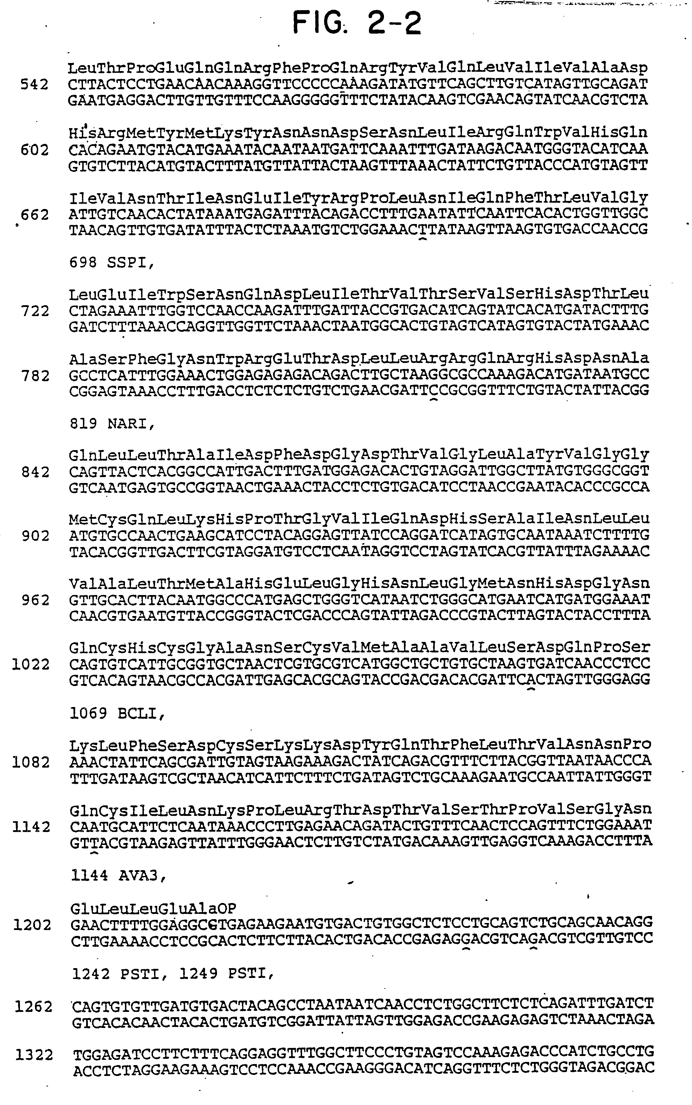

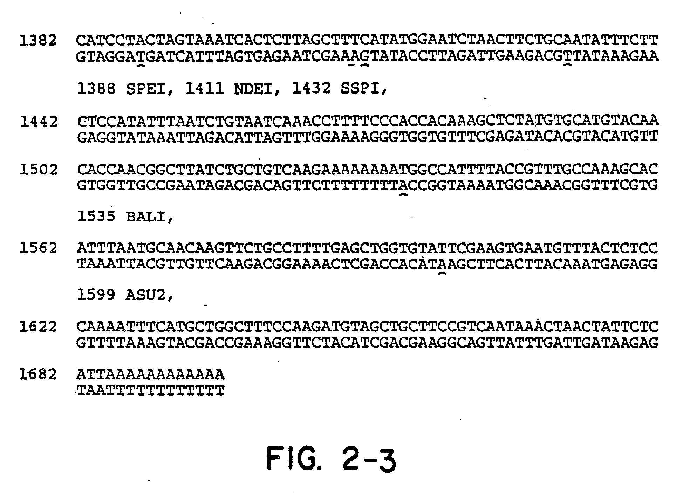

Further amino acid sequencing of native protein provided an amino acid sequence for the mature fibrolase protein differing by two amino acids from the sequence predicted from Fib 51 (Figures 2 and 4). The six differences are (numbering begins at amino acid 185 of Figure 2): 123 (Pro-----Ser) and 170 (Val-----Met).

This sequence is sometimes referred to herein as the "corrected native sequence".

It is further expected that fibrolases from other Crotalidus species may also vary intraspecies as well as varying from species to species but will exhibit significant homology (i.e., 30% or more identity, more usually 50% or more identity, in amino acid sequence to the southern copperhead fibrolase sequences described herein). Fibrolase DNA of Crotalidae of species other than southern copperhead fibrolase may be identified and isolated as described below. Amino acid sequences may be deduced from that DNA.

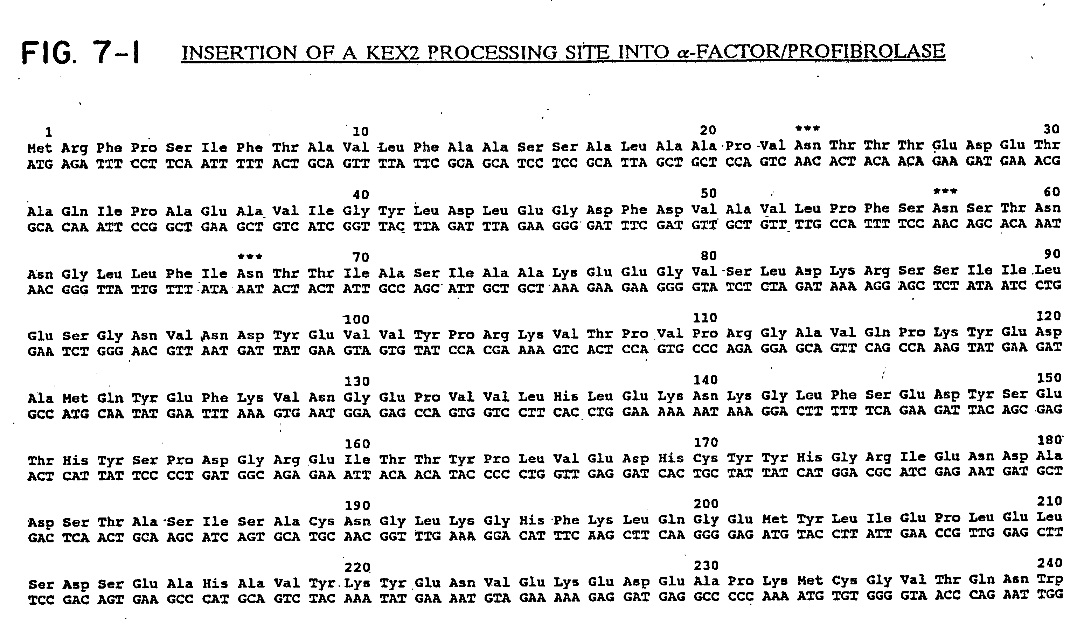

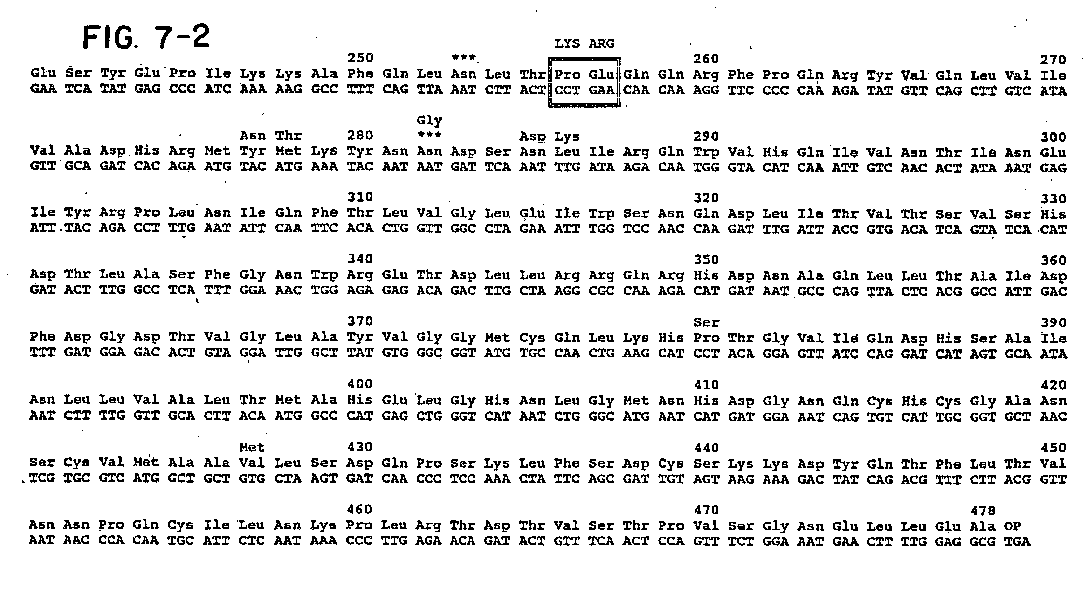

The recombinant southern copperhead DNA of the invention encodes at least amino acids 192 to 393 of the Fib 48 sequence of Figure 4, or the corresponding amino acids of the Fib 51 sequence of Figure 4 (amino acids 186-387 of the Fib 51 sequence), or the corrected native amino acid sequence shown in Figure 7, and analogs of those amino acid sequences which are substantially homologous and functionally equivalent thereto. Based on the sequences of clones pSVG-48 and pSVG-51 shown in Figures 1 and 2 and amino acid sequencing of native southern copperhead fibrolase the structure of southern copperhead fibrolase is believed to be that of a prepropolypeptide with the first 190-191 amino acids of the Fib 48· sequence being a leader sequence and the mature protein beginning at the Gln at position 190 or the Gln at position 191 (Figure 1) or at 185/186 in the Fib 51 sequence (Figure 2). It is further believed that the carboxy terminus of the prepropolypeptide is processed to remove the final 18 amino acids. Mass spectrophotometric analysis of peptides derived from mature native southern copperhead fibrolase indicates the amino terminus is a cyclized glutamine residue with some molecules starting at the Gln at 185 (i.e., pGlu-Gln-Arg/Phe-...) and other molecules starting at the Gln at 186 (i.e., pGlu-Arg/Phe-...) of Figure 2. Reaction of mature native fibrolase with DTNB indicated there are no free sulfhydryl groups in southern copperhead fibrolase meaning there are three disulfide bonds in the molecule. The term "substantially homologous" as used herein intends to include such variations polymorphic and/or allelic variations as well as other variations that do not destroy the fibrolase activity of the molecule. In general, the homology in amino acid sequence will be at least about 70%, more usually at least about 75%. The term "functionally equivalent" intends that the sequence of the analog defines a protein having the biological activity of fibrolase (as measured by the azocasein or fluorometric assays described in the examples). The sequence may include a portion or all of the leader sequence (amino acids 1-190/191 and all or a portion of the processed carboxy terminus of the prepropolypeptide (amino acids 393-411 of Fib 48 or the corresponding amino acids of Fib 51).

The recombinant fibrolase DNA may be genomic, cDNA or synthetic DNA. By way of example, the sequences shown in Figures 1 and 2 were obtained from a cDNA library prepared from mRNA obtained from southern copperhead venom glands. The library was screened with cDNA probes based on abundant mRNA sequences and clones showing strong hybridization were selected. Identified fibrolase clones may in turn be used to screen genomic or cDNA southern copperhead libraries to obtain allelic or polymorphic variants of the fibrolase genes shown in the drawings or to screen libraries of other Crotalidus species to identify the fibrolase genes contained therein. A DNA sequence encoding the "native" protein sequence may be made by site specific mutagenis of clone 51 cDNA or by other conventional methods. The general procedures used to prepare probes based on the identified clones, to prepare other libraries, and to screen those libraries for Crotalidus fibrolase sequences are known in the art and do not require elaboration. Based on the amino acid sequences deduced from the illustrated DNA sequences, synthetic genes encoding fibrolase may be prepared in vitro by synthesizing individual overlapping complementary oligonucleotides and filling in single stranded nonoverlapping portions using DNA polymerase in the presence of the deoxyribonucleotide triphosphates. For expression in a particular organism, it may be desirable to use a synthetic DNA sequence that employs codons preferred by the particular host in which the DNA is expressed. Mutations of the genes may be made by site specific mutagenesis. Such mutations may be used to make fibrolase analogs.

3. Cloning of Fibrolase DNA

The fibrolase DNA can be cloned into any suitable replicon to create a vector, and thereby be maintained in a composition which is substantially free of vectors that do not contain the fibrolase gene (e.g., other clones derived from the library). Numerous cloning vectors are known to those of skill in the art, and the selection of an appropriate cloning vector is a matter of choice. Examples of vectors for cloning and host cells which they can transform include the bacteriophage λ (E. coli), pBR322 (E. coli), pACYC 177 (E. coli), pKT230 (gram-negative bacteria), pGV1106 (gram-negative bacteria), pLAFR1 (gram-negative bacteria), pME290 (non-E. coli gram-negative bacteria), pHV 14 (E. coli and Bacillus subtilis), pBD9 (Bacillus), pIJ61 (Streptomyces), pUC6 (Streptomyces), actinophage φC31 (Streptomyces), YIp5 (Saccharomyces), YCp19 (Saccharomyces), YEp24 and YEp13 (Saccharomyces), and bovine papilloma virus (mammalian cells).

4. Expression of Fibrolase DNA

The polynucleotide sequence encoding the fibrolase polypeptide is expressed by inserting the sequence into an appropriate replicon thereby creating an expression vector, and introducing the resulting expression vector into a compatible host.

In creating an expression vector the sequence encoding the fibrolase polypeptide is located in the vector with the appropriate control sequences. The positioning and orientation of the coding sequence with respect to the control sequences is such that the coding sequence is transcribed under the control of the control sequences: i.e., the promoter will control the transcription of the mRNA derived from the coding sequence; and the ribosomes will bind at the ribosomal binding site to begin the translational process; and the stop codon used to terminate translation will be upstream from the transcriptional termination codon. Commonly used prokaryotic control sequences include such commonly used promoters as the β-lactamase (penicillinase) and lactose (lac) promoter systems (Chang et al, Nature (1977) 198:1056) and the tryptophan (trp) promoter system (Goeddel et al, Nucleic Acids Res (1980) 8:4057) and the lambda-derived $P_L$ promoter and N-gene ribosome binding site (Shimatake et al, Nature (1981) 292:128). Control sequences for yeast vectors include promoters for the synthesis of glycolytic enzymes (Hess et al, J Adv Enzyme Reg (1968) 7:149; Holland et al, Biochemistry (1978) 17:4900). Additional promoters known in the art include the promoter for 3-phosphoglycerate kinase (Hitzeman et al, J Biol Chem (1980) 255:2073). Other promoters, which have the additional advantage of transcription controlled by growth conditions and/or genetic background are the promoter regions for alcohol dehydrogenase 2 (ADH2), isocytochrome C, acid phosphatase, degradative enzymes associated with nitrogen metabolism, the alpha factor system and enzymes responsible for maltose and galactose utilization. It is also believed transcription terminator sequences are desirable at the 3' end of the coding sequences. Such terminators are found in the 3' untranslated region following the coding sequences in yeast-derived genes. Expression vectors for mammalian cells such as VERO, Hela or CHO cells, ordinarily include promoters and control sequences compatible with such cells as, for example, the commonly used early and late promoters from Simian Virus 40 (SV40) (Fiers et al, Nature (1978) 273:113), or other viral promoters such as those derived from polyoma, Adenovirus 2, bovine papilloma virus, or avian sarcoma viruses. The controllable promoter, hMTII (Karin, M., et al, Nature (1982) 299:797-802) may also be used.

In addition to control sequences, it may be desirable to add regulatory sequences which allow for regulation of the expression of the fibrolase gene relative to the growth of the host cell. Examples of regulatory systems are those which cause the expression of a gene to be turned on or off in response to a chemical or physical stimulus, including the presence of a regulatory compound. In prokaryotic systems these would include the lac and trp operator systems. In eukaryotic systems induction can occur in methallothionein genes with heavy metals and the Mouse Mammary Tumor Virus (MMTV) system with steroids. In these cases, the sequence encoding the fibrolase polypeptide would be placed in tandem with the regulatory element.

There are also selective elements which give rise to DNA amplification which in turn can result in higher levels of specific protein production. In eukaryotic systems these include the dihydrofolate reductase gene (dhfr) which is amplified in the presence of methotrexate, and adenosine deaminase (ADA) in the presence of deoxycorfomycin. In these cases the sequence encoding the fibrolase polypeptide may either be present on the same plasmid or merely be cotransfected together with the selectable element to allow for integration within the host cell genome near each other.

Other types of regulatory elements may also be present in the vector, i.e., those which are not necessarily in tandem with the sequence encoding fibrolase. An example is the SV40 enhancer sequence, which, by its mere presence, causes an enhancement of expression of genes distal to it.

Modification of the sequence encoding fibrolase, prior to its insertion into the replicon, may be desirable or necessary, depending upon the expression system chosen. For example, in some cases, it may be necessary to modify the sequence so that it may be attached to the control sequences with the appropriate orientation, i.e., to maintain the reading frame. In some cases, it may be desirable to add sequences which cause the secretion of the polypeptide from the host organism, with subsequent cleavage of the secretory signal. The techniques for modifying nucleotide sequences utilizing cloning are well known to those skilled in the art. They include, e.g., the use of restriction enzymes, of enzymes such as Bal31 to remove excess nucleotides, and of chemically synthesized oligonucleotides for use as adapters, to replace lost nucleotides, and in site directed mutagenesis.

The appropriately modified sequence encoding the fibrolase polypeptide may be ligated to the control sequences prior to insertion into a vector. Alternatively, the coding sequence can be cloned directly into an expression vector which already contains the control sequences and an appropriate restriction site. For expression of the fibrolase polypeptide in prokaryotes and in yeast, the control sequences will necessarily be heterologous to the coding sequence. In cases where the fibrolase gene is to be expressed in cell lines derived from vertebrates, the control sequences may be either heterologous or homologous, depending upon the particular cell line.

In experimentation with various expression vectors--integrated, nonintegrated, intracellular (internal), extracellular (secretory)--carried out to date, yeast expression vectors in which the S. cerivisiae α-factor leader sequence directs secretory expression of a profibrolase gene encoding the corrected native fibrolase sequence and having an Lys Arg (KR) processing site at the pro-mature junction of the profibrolase coding region have given the highest yields of active mature fibrolase. Constructs using both nonregulatable promoters (e.g., GAPDH) and regulatable promoters (e.g. ADH2/GAPDH) were made. Use of a regulatable promoter may be desirable to overcome possible toxic effects from large amounts of fibrolase during cell growth. A summary of these constructs is provided in the Examples, infra.

Depending on the host cell used, transformation is done using standard techniques appropriate to such cells. The calcium treatment employing calcium chloride, as described by Cohen, S.N., Proc Natl Acad Sci (USA) (1972) 69:2110, or the $RbCl_2$ method described in Maniatis et al, Molecular Cloning: A Laboratory Manual (1982) Cold Spring Harbor Press, p. 254 and Hanahan, D., J Mol Biol (1983) 166: 557-580 may be used for prokaryotes or other cells which contain substantial cell wall barriers. For mammalian cells without such cell walls, the calcium phosphate precipitation method of Graham and van der Eb, Virology (1978) 52:546, optionally as modified by Wigler, M., et al, Cell (1979) 16:777-785 may be used. Transformations into yeast may be carried out according to the method of Beggs, J.D., Nature (1978) 275:104-109 or of Hinnen, A., et al, Proc Natl Acad Sci (USA) (1978) 75:1929 or Ito et al, J Bacteriology 153:163.

Transformed cells are then grown under conditions which permit expression of the fibrolase gene and, if appropriate, processing into the mature protein. Because the gene is expressed in heterologous organisms/cells (i.e., not snake), the protein is free of the other snake proteins with which it is associated in southern copperhead venom. This recombinant fibrolase is also homogeneous and lacks allelic/polymorphic variations typically found in protein isolated from pooled snake venom. The thus synthesized recombinant fibrolase is then isolated from the host cells and purified. If the expression system secretes the fibrolase into the growth media, the fibrolase is isolated directly from the media. If the recombinant fibrolase is not secreted, it is isolated from cell lysates. The selection of the appropriate growth conditions and recovery methods are within the skill of the art. With regard to purification, see for instance, U.S. Patent No. 4,610,879.

## 5. Use and Administration of Recombinant Fibrolase

Recombinant fibrolase prepared according to the invention may be formulated and administered to vertebrates, particularly mammals including humans, for treatment of thromboembolic conditions in the same manner as fibrolase isolated from venom (see U.S. Pat. No. 4,610,879 and EPA Publication No. 0020780 in this regard). The fibrolase will typically be formulated with a pharmaceutically acceptable injectable carrier such as physiological saline, Ringer's solution and the like for injection into circulation. Mildly hydrophilic polymeric substances such as albumins, polyvinylalcohol, polyethylene glycol, and the like may be added to stabilize the fibrolase. The concentration of fibrolase in the injectable will normally be in the range of 0.01 to 1 mg/ml. The patient will normally be infused with a dose of about 0.1 to 80 mg fibrolase for adult humans.

## Examples

The following examples further describe the isolation of DNA encoding fibrolase and the expression of that DNA in yeast to produce recombinant fibrolase.

In the following, "digestion" refers to the enzymatic cleavage of DNA by restriction endonucleases. Restriction endonucleases commonly referred to as restriction enzymes are well characterized and commercially available and used in accordance with manufacturer's specifications. Digestion with restriction enzymes is frequently followed by treatment with alkaline phosphatase according to manufacturer's specifications to remove the terminal 5' phosphates, thus preventing self ligation of a vector having two compatible ends.

"Fill in" refers to the enzymatic process of creating blunt ends by repairing overhanging ends generated by certain restriction enzymes. The repair is a DNA polymerase I large fragment (Klenow) and deoxynucleotide triphosphates and is used according to manufacturer's specifications.

Gel isolation of a DNA restriction fragment refers to the recovery of a specific fragment, electrophoretically separated on either an agarose gel or polyacrylamide gel (depending on size of fragment), by either electroelution or melting and extraction of gel slice.

All DNA manipulations are done according to standard procedures. See Maniatis et al, Molecular Cloning, Cold Spring Harbor Lab., 1982. All enzymes used are obtained from commercial sources and used according to the manufacturer's specifications.

### 1. RNA Isolation from Southern Copperhead Venom Glands

The method used to isolate RNA from snake venom glands utilizes guanidinium thiocyanate as a chaotropic agent during tissue homogenization, direct precipitation of RNA from the guanidinium solution by LiCl followed by purification of the RNA from residual DNA and protein by successive urea-LiCl washes.

Southern copperhead snakes (Agkistrodon c. contortrix) were obtained from Zooherp, Inc. Three snakes were killed with a lethal dose of Nembutol and the six venom glands were dissected free of the surrounding muscle. They were frozen in liquid nitrogen and the pulverized tissue was homogenized in 8 ml of lysis buffer containing 5 M guanidinium thiocyanate, 100 mM Tris•Hcl (pH 7.5), 10 mM EDTA (pH 8.0) and 14% (v/v) beta-mercaptoethanol using a Tekmar Tissumizer for one min at room temperature. After dissolution, the homogenate was clarified by centrifugation (10,000 X g, 4°C, 10 min). The RNA was precipitated (16-24 hr, 4°C) by adding 5.5 volumes of 4 M LiCl. The RNA and some protein and DNA was then pelleted by centrifugation (10,000 X g, 4°C, 30 min). The pellet was mechanically resuspended in 5 ml of a solution of 3 M LiCl containing 4 M urea by using a pasteur pipet sealed at the end, and then vortexed vigorously for about one min. The volume of the suspension was adjusted to 30 ml and the precipitate collected by centrifugation as described above. This procedure was repeated 2 times. The RNA pellet was finally dissolved in 2.5 ml of 1% sodium dodecyl sulfate (SDS). One volume of phenol (equilibrated with 50 mM Tris•Hcl, pH 8.0, 1 mM EDTA and 0.1% beta-mercaptoethanol) was added and the mixture vortexed vigorously for about one min. An equivalent volume of chloroform:isoamyl alcohol (24:1) was added and the solution vortexed one min. The mixture was centrifuged (4,000 X g, 4°C, 20 min); the aqueous phase was extracted with chloroform:isoamyl alcohol and centrifuged (4,000 X g, 4°C, 10 min). The RNA was precipitated (16-24 hr, -20°C) from the aqueous phase by adding 1/10 volume of 2 M potassium acetate (pH 5.0) and 3 volumes of absolute ethanol. The precipitated RNA was collected by centrifugation (10,000 X g, 4°C, 30 min) and dissolved in 0.5 ml of diethylpyrocarbonate-(DEP)-treated water and poly(A)+ RNA was prepared as described by Maniatis et al. The concentration of the poly(A)+ RNA was calculated based on an extinction coefficient at 260 nm of 25 per mg of RNA. The RNA was precipitated as described above and stored at -20°C.

### 2. Construction of cDNA Library from Southern Copperhead Venom Gland RNA

Double-stranded cDNA was prepared from Southern copperhead venom gland poly(A)+ RNA essentially as described by Gubler, U. and Hoffman, B.J., Gene (1983) 25:263-269. After methylation of the internal EcoRI sites and the addition of EcoRI linkers, the cDNA was ligated into the EcoRI site of λgt10. These steps are described in detail below:

A. For first strand synthesis, 3 $\mu$g of poly(A) + RNA in 6 $\mu$l of DEP-treated water was used in a total volume of 20 $\mu$l. The final mixture contained 2 $\mu$l of 0.1 M dithiothreitol (DTT), 1 $\mu$l of RNasin (Promega Biotec, diluted to 10 units/$\mu$l in 10 mM DDT), 2 $\mu$l of 0.1 M MgCl$_2$, 1 $\mu$l of 1M Tris•Hcl (pH 8.3), 2 $\mu$l of 1 mg/ml oligo(dT)12-18 (P-L Biochemicals), 2 $\mu$l of 10 mM each d(ACGT)Tps (P-L Biochemicals), 1 $\mu$l of [$^{32}$p] dCTP (Amersham, 3000 Ci/mmole), 1 $\mu$l of 80 mM sodium pyrophosphate and 2 $\mu$l of reverse transcriptase (Molecular Genetics Resources). The reactants were combined on ice, incubated for 2 min at room temperature and then for 60 min at 45° C. The reaction was stopped by adding 2 $\mu$l of 0.5 M EDTA (pH 7.0) and then phenol/chloroform/isoamyl alcohol (25/24/1) extracted. The organic phase was extracted with an equal volume of TE (10 mM Tris•Hcl, pH 8.0, 1 mM EDTA), the aqueous phases were combined and the first strand cDNA was precipitated by adding 16 $\mu$l of 7.5 M ammonium·acetate and 120 $\mu$l of absolute ethanol. The pellet of precipitated nucleic acid was collected by microcentrifugation, dried in a Savant Speed-Vac Concentrator, dissolved in 50 $\mu$l of H$_2$O and precipitated by adding 20 $\mu$l of 7.5 M ammonium acetate and 150 $\mu$l of absolute ethanol. This procedure was repeated a third·time, the pellet was rinsed in absolute ethanol and dissolved in 71 $\mu$l of DEP-treated water.

B. For second strand cDNA synthesis, 71 $\mu$l of first-strand cDNA was used in a total volume of 100 $\mu$l that included 2 $\mu$l of 1 M Tris•Hcl (pH 7.5), 5 $\mu$l of 100 mM MgCl$_2$, 1 $\mu$l of 1 M ammonium sulfate, 10 $\mu$l of 1 M KCl, 1.25 $\mu$l of 4 mg/ml bovine serum albumin, 5 $\mu$l of 1 mM each of d(ACGT)TPs, 1 $\mu$l of [$^{32}$P]-dCTP (Amersham, 3000 Ci/mmole), 1.5 $\mu$l of RNase H (Bethesda Research Laboratories) and 2.3 $\mu$l (23 units) of DNA polymerase I (New England Biolabs). The reaction was incubated for 1 h at 14° C and then for 1 h at room temperature. It was terminated by the addition of 4 $\mu$l of 0.5 M EDTA (pH 7.0) and extracted with an equal volume of phenol/chloroform/isoamyl alcohol. The double-stranded cDNA was precipitated by the addition of 40 $\mu$l of 7.5 M ammonium acetate and 300 $\mu$l of absolute ethanol. The pellet was reprecipitated two times as described in step I. Finally, the pellet of cDNA was rinsed in absolute ethanol, dried and dissolved in 25 $\mu$l of DEP-treated water.

C. To fill in the ends of the cDNA so that chemically synthesized linkers could be ligated to them, 7.7 $\mu$l of DEP-treated water was added to the 25 $\mu$l of double-stranded cDNA. The final reaction volume was 50 $\mu$l and included 5 $\mu$l of (0.5 M Tris•Hcl, pH 8.0, 60 mM MgCl$_2$ and 250 mM KCl), 5 $\mu$l of 10 mM DTT, 5 $\mu$l of 1 mM each d(ACGT)TPs, 1.3 $\mu$l of 4 mg/ml BSA and 1 $\mu$l (5 units) of T4 DNA polymerase (Bethesda Research Laboratory). The reaction was incubated at 37° C for 30 min and terminated by the addition of 2 $\mu$l of 0.5 M EDTA (pH 7.5). The solution was heated for 10 min at 70° C and the nucleic acid precipitated by the addition of 20 $\mu$l of 7.5 M ammonium acetate and 150 $\mu$l of ethanol. The precipitate was collected, reprecipitated once, rinsed and dried as described in step I. The DNA was dissolved in 27.5 $\mu$l of TE.

D. To protect internal EcoRI sites within the DNA from subsequent cleavage, the DNA was modified by EcoRI methylase. To 27.5 $\mu$l of DNA was added 10 $\mu$l of 5X buffer (0.5 M Tris•Hcl, pH 8.0, 5 mM EDTA), 5 $\mu$l of 4 mg/ml BSA, 5 $\mu$l of 100 $\mu$M SAM (S-adenosyl-L-methionine, Sigma) and 2.5 $\mu$l (50 units) EcoRI methylase (New England Biolabs). The reaction was incubated at 37° C for 30 min and then heated at 70° C for 10 min. The methylated cDNA was precipitated by adding 20 $\mu$l of 7.5 M ammonium acetate and 150 $\mu$l of absolute ethanol. The precipitate was collected, reprecipitated twice, rinsed and dried as described in step A.

E. EcoRI dodecamer linkers were kinased in a total volume of 50 $\mu$l as follows; 15 $\mu$l (1.5 mmoles) of linker solution (Collaborative Research), 5 $\mu$l of 10X kinase buffer (700 mM Tris•Hcl, pH 7.5, 100 mM MgCl$_2$, 50 mM DTT), 5 $\mu$l of 10 mM ATP, 22 $\mu$l of H$_2$O and 3 $\mu$l (15 units) of T4 polynucleotide kinase (New England Biolabs). The reaction was incubated at 37° C for 2 h.

F. To ligate the kinased EcoRI linkers to the methylated cDNA, 10 $\mu$l of water was added to the dried cDNA from step D followed by 10 $\mu$l of linkers from step E, 1.5 $\mu$l of 10 mM ATP, 1.5 $\mu$l of 10X ligase buffer (0.5 M Tris•Hcl pH 8.0, 0.1 M MgCl2, 0.2 M DTT) and 2 $\mu$l of T4 DNA ligase (New England Biolabs). The total reaction of 25 $\mu$l was incubated at 14° C overnight.

G. The cDNA, the T4 DNA ligase was inactivated by heating the reaction from step F for 15 min at 70° C. To the mixture was added 70 $\mu$l of water, 10 $\mu$l of 10X TMN (100 mM Tris•Hcl, pH 7.5, 100 mM MgCl$_2$ and 1.0 M NaCl) and 5 $\mu$l (100 units) of EcoRI. The 100 $\mu$l reaction was incubated at 37° C for 3 h to remove excess linkers from the cDNA and then phenol/chloroform/isoamyl alcohol extracted. One-tenth volume of 5 M NaCl was added to the aqueous phase and the "trimmed" cDNA was fractionated from excess linkers by gel filtration on a 0.7 X 18 cm Sepharose CL4B column. The elution buffer was 0.4 M NaCl. 10 mM Tris•Hcl, pH 8.0, 1 mM EDTA. The radioactivity of the fractions was determined and the peak of cDNA pooled and precipitated one time with 2 volumes ethanol as described in step A then precipitated.

H. To prepare the λgt10 vector for cloning the cDNA, 25 μg of λgt10 was digested with EcoRI. The final reaction volume was 250 μl and contained 1X TMN and 100 units of EcoRI. The enzyme digestion was carried out at 37°C for 2 h and was then extracted with chloroform. The linearized λgt10 was precipitated one time with ethanol as described in step A. The dried DNA was dissolved in 100 μl of TE.

I. To ligate the vector DNA to the cDNA, 12 μl (3 μg) was added to the ethanol-precipitated EcoRI-linked cDNA (step G). The mixture was dried and 7 μl of water was added followed by 1 μl of 10X ligase buffer and 1 μl of 10 mM ATP. The mixture was incubated at 37°C for 5 min, chilled to 4°C and 1 μl of T4 DNA ligase was then added. After incubation at 14°C overnight, the ligation mixture was stored at -20°C.

J. To package the recombinant DNA from step I into phage particles and plate them using E. coli strain C600Δhfl as a host, one-third (3 μl) of the ligation mixture was treated using Gigapack-plus according to the manufacturer's (Stratagene) instructions. Two hundred thousand recombinant phage were obtained.

## 3. Screening of the Snake Venom Gland cDNA Library

One thousand recombinant phage from the southern copperhead snake venom gland cDNA library were plated onto Luria agar using E. coli host strain C600Δhfl according to the manufacturer's (Strategene) instructions. The transfer of phage DNA to nitrocellulose filters (phage lifts) and the subsequent hybridization of the filters with $^{32}$P-labeled single stranded (first strand) cDNA probe were done essentially as described by Maniatis et al. This strategy was selected since fibrolase is an abundant protein in snake venom and, therefore, the mRNA encoding this enzyme is expected to also be abundant.

The plaque lifts were done in duplicate, and the filters preannealed overnight at 42°C in 50% formamide, 5% SSC, 2x Denhardt's, 50 mM sodium phosphate, pH 6.5, 0.25% SDS and 100 μg/ml denatured salmon testes DNA. The filters were then hybridized with 250,000 cpm/ml of the $^{32}$P-labeled stranded cDNA probe (boiled for 10 min in 0.4 N NaOH) made from the poly(A) + RNA using the procedure described previously. Hybridization was done at 42°C overnight in preanneal solution that also contained 10% (w/v) sodium dextran sulfate. Washing of the filters was done in 2x SSC, 0.1% SDS for 30 min at room temperature, followed by 30 min at 50°C. The autoradiogram was exposed with an intensifying screen at 80°C overnight.

Sixty of the strongly hybridizing plaques were removed by coring and the phage eluted into PSB (100 mM NaCl, 10 mM Tris, pH 7.5, 10 mM MgCl2, 500 mg/l gelatin). By plating appropriate dilutions of the phage stock eluate and screening by hybridization as described, an isolated bacteriophage colony was obtained for six of the original plaque.

Lambda DNA was isolated from each clone and the EcoRI-EcoRI cDNA inserts were purified away from the vector.

Restriction digests were done according to manufacturer's (New England Biolabs) instructions on the EcoRI fragments using PstI and HindIII separately. The results of the restriction analyses are shown in Table 1. The restriction analyses show that of the 6 clones screened, there are 3 distinct classes. Two of these (SVG-48 and SVG-51) were completely sequenced. The sequences are shown in Figs 1 and 2, respectively.

Table 1

| Clone | Eco RI Fragments (kb) | Pst I Fragments (kb) | Hind III Fragments (kb) |
|---|---|---|---|
| SVG-6 | 1.1 | 0.5-, 0.6 + | 1.1 |
| SVG-8 | 1.8 | 0.2, 0.6, 1.1 | 0.4, 1.4 |
| SVG-20 | 1.3 | 0.5, 0.8 | 1.3 |
| SVG-23 | 1.8 | 0.2, 0.5-, 1.1 | 0.4, 1.4- |
| SVG-48 | 1.8 | 0.2, 0.6, 1.1 | 0.4, 1.4 |
| SVG-51 | 1.8 | 0.2, 0.5, 1.1 | 0.4, 1.4 |

## 4. Intracellular Expression of Fibrolase Protein

The following describes the intracellular expression of the fibrolase protein in yeast under the control of the regulatable promoter ADH2-glyceraldehyde-3-phosphate-dehydrogenase (GAPDH or GAP) and GAPDH terminator.

## A. Construction of a Yeast Expression Vector for Fibrolase:

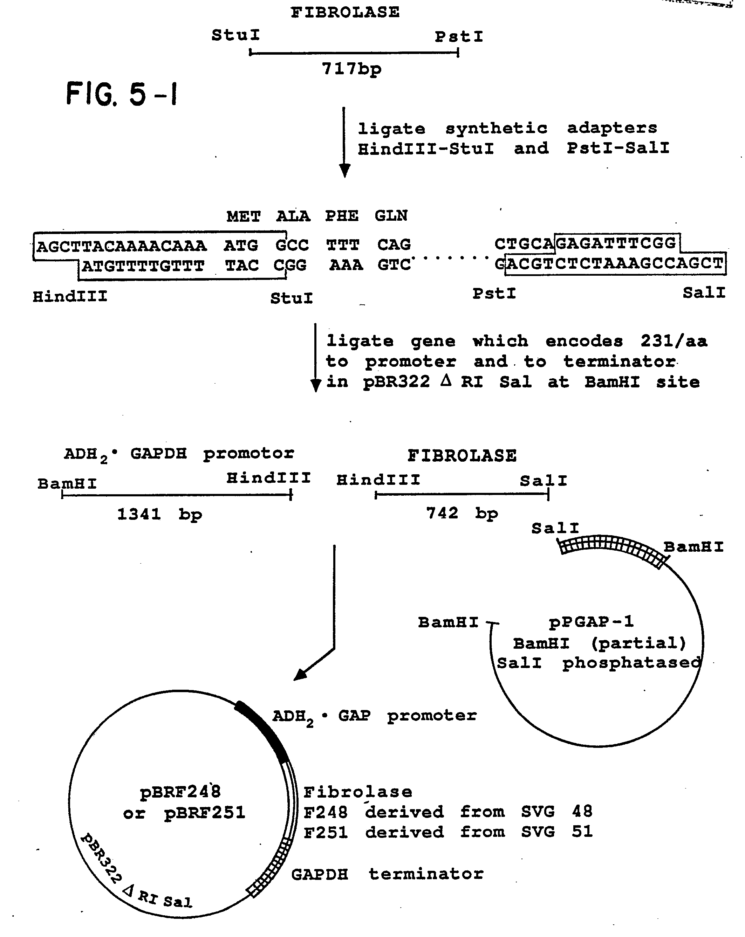

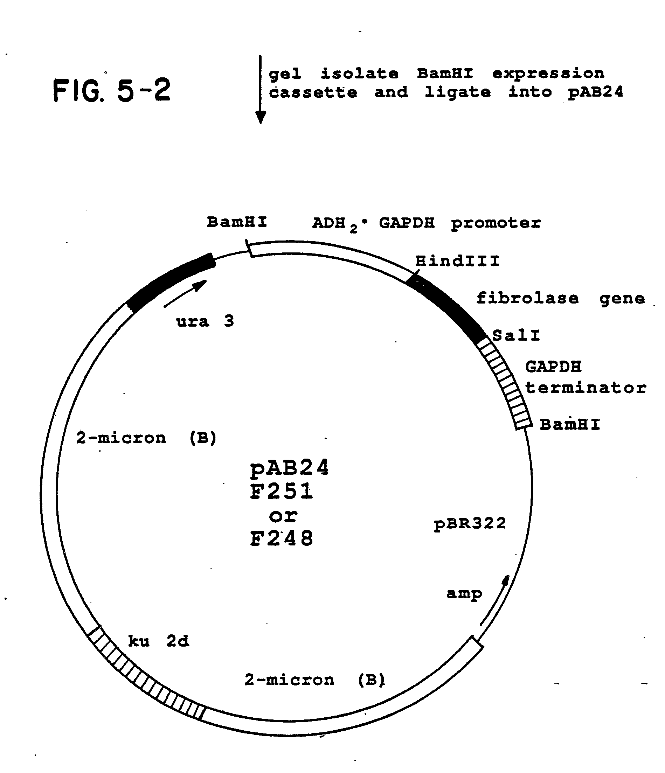

The scheme used to prepare the yeast expression vector is shown in Figure 5.

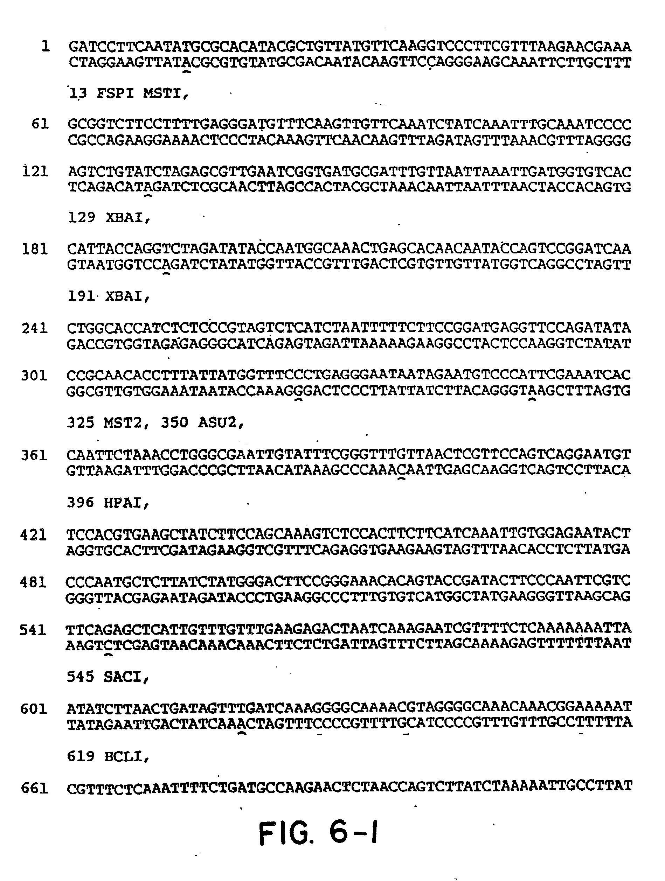

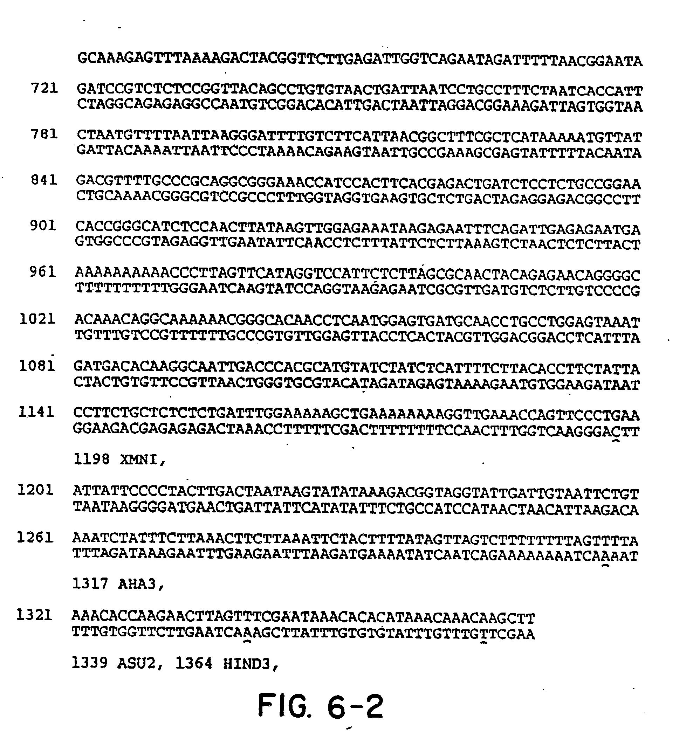

SVG 48 (or SVG 51) was digested with StuI and PstI and the resulting 717 bp StuI/PstI fragment was gel isolated. That fragment was ligated to the HindIII/StuI and PstI/SalI synthetic adaptors shown in Figure 5. The resulting 742 bp SalI/HindIII fibrolase gene encoding 231 amino acids was ligated to a 1341 bp ADH2-GAPDH promoter fragment (Figure 6). The resulting ligation product was ligated into the plasmid pPGAP-1 which had been cut with BamHI and SalI. pPGAPI is a yeast expression cassette vector which has a polyrestriction site linker between the GAPDH terminator and a truncated GAPDH promoter region. The polyrestriction site contains the recognition sites for NcoI, EcoRI, and SalI, and the cassette is excisable as a BamHI fragment. The preparation of pPGAPI is described in EPO 0 164 556 and Travis, J., et al, J Biol Chem (1985) 260(7):4384-4389. In both references pPGAPI is referred to pPGAP. The resulting plasmid was called pBRF248 (or pBRF251).

pBRF248 (or pBRF251) was digested with BamHI and the resulting BamHI cassette containing the ADH2-GAPDH promoter, fibrolase structural gene, and GAPDH terminator was gel purified and ligated in plasmid pAB24 which had been cut with BamHI. pAB24 is a yeast shuttle vector which contains the complete 2 μ sequences (Broach, In: Molecular Biology of the Yeast Saccharomyces, 1:445, Cold Spring Harbor Press (1981)) and pBR322 sequences. It also contains the yeast URA3 gene derived from plasmid YEp24 (Botstein et al, Gene (1979) 8:17) and the yeast LEU2$^d$ gene derived from plasmid pCI/I (described in European Patent Application publication no. EPO116201). Insertion of the expression cassette was in the BamHI site of pBR322, thus interrupting the gene for bacterial resistance to tetracycline. The resulting plasmid was designated pAB24F248 (or pAB24F251).

Plasmid pAB24F248 (or pAB24F251) was transformed into yeast strain S. cerevisiae 2150-2-3 Mata, adel, leu2-04, [cir°] (strain from the collection of L. Hartwell, University of Washington, Seattle) and plated on leu⁻ 8% glucose. Transformants were streaked on leu⁻ 8% glucose to obtain single colonies which were patched onto leu⁻ 8% glucose plates. Cells from 8 patches were grown for ~24 hr in leu⁻ 8% glucose minimal media and then inoculated (1:25 v/v) in YEP 1% glucose and grown for 96 hr at 30° C. Samples (2.5 OD units) were collected for gel analysis at 24, 48, 72, and 96 hr.

Cell samples were boiled in SDS sample buffer (0.0675M Tris•Cl pH 6.8, 3% SDS, 10% glycerol) containing 50 mM DTT and run on 12.5% SDS-acrylamide gels. Coomassie staining of the gels showed a prominent band in the predicted 25.9 kd size range. This band accounted for ~20% of the total cell protein and was not found in pAB24 control extracts. It was seen in samples collected at each time point.

Western blot analyses were carried out on eight 48 hr samples of pAB24F248 and pAB24F251 transformants using rabbit anti-fibrolase sera. The antisera reacted strongly with the 25.9 kd band in all eight samples.

There were also lower molecular weight bands that reacted with the antisera, suggesting some degradation of the fibrolase in the yeast.

## B. Construction of Yeast Expression Vector Encoding Corrected Native Fibrolase Sequence

Using pAB24F251 as a starting plasmid an internal expression plasmid was constructed which encodes the corrected native sequence (i.e., the authentic amino terminal sequence of mature southern copperhead fibrolase preceded by a Met residue [i.e., (Met) Gln-Gln-Arg-Phe-Pro-Gln-Arg....] and the amino acid changes at positions 123 and 170 (described supra) of the mature protein). Sequence changes were made by conventional in vitro mutagenesis techniques. This plasmid was designated pAB24F751.

S. cerevisiae cells transformed with pAB24F751 were observed to express a high level of protein corresponding in size to native fibrolase. Testing of cell lysates by the azocasein hydrolysis assay, however, showed no activity.

## 5. Construction of Plasmids For Secretory Expression of Mature Fibrolase and Profibrolase

A series of plasmids were made to direct the secretory expression of mature fibrolase or profibrolase (Profib). These plasmids are summarized in the following table.

| Plasmid | Vector | Promoter | Leader | Fibrolase |
|---------|--------|----------|--------|-----------|
| pKS308 | pAB24 | GAP | αF | Mature Fibrolase |
| pKS311 | pAB24 | GAP | αF | Profib |
| pKS313 | pAB24 | ADH2/GAP | αF | Pro(KR)fib |
| pKS314 | pAB24 | GAP | αF | Pro(KR)fib |
| pKS316 | pAB24 | ADH2/GAP | αF(ΔKR) | Pro(KR)fib |
| pKS317 | pAB24 | GAP | αF(ΔKR) | Pro(KR)fib |
| pKS359 | pAB38 | GAP | αF | Profib |
| pKS362 | pAB38 | GAP | αF | Pro(KR)fib |
| pKS365 | pAB38 | GAP | αF(ΔKR) | Pro(KR)fib |

GAP = glyceraldehyde-3-phosphate-dehydrogenase

ADH2 = alcohol degydrogenase 2

αF = S. cerevisiae alpha factor

KR = Lys-Arg processing site

ΔKR = minus Lys-Arg processing site

The fibrolase gene of pKS308 was derived from pAB24F751 and thus encodes the corrected native sequence. The profibrolase gene used in pKS311 was derived from Fib 51 and thus does not have the amino acid changes of the corrected native sequence but does include the 18 amino acid C-terminal extension of the Fib 51 sequence. A K. lactis counterpart of pKS311 (designated pKS359) was made using the K. lactis vector pAB38 instead of pAB24. pAB38 is a derivative of pUC18 (Yanisch-Perron et al., Gene (1985) 33:103) and was prepared as follows. pUC18 was cleaved with HindIII and a 1.2 kb HindIII fragment containing the URA3 gene (Botstein et al., Gene (1979) 8:17) was ligated into pUC18. Further, a PKDI (Falcone et al., Plasmid (1986) 15:248) EcoRI insert from Kluyveromyces drosophilarum was ligated into the EcoRI site of pUC18. Finally, the vector was cleaved with NarI/HindIII (partial), filled in with Klenow, and religated to yield pAB38. The S. cerevisiae α-factor leader sequence and its incorporation into vectors is described in EPO Publication 116,201. The ADH2/GAP promoter and its incorporation into vectors is described in EPO Publication 164,556.

Expression of the pKS311 and pKS359 constructs in transformed S. cerevisiae and K. lactis yielded a substantial amount of immunoreactive species having the size of profibrolase. It thus appeared that a substantial portion of profibrolase was not being processed into the mature protein. Constructs were thus made in which a Lys-Arg processing site was introduced using synthetic oligonucleotides at the pro-mature junction which should be recognized by the yeast KEX2-encoded protease, the same enzyme that carries out the cleavage at the junction of the α-factor leader. Figure 7 shows the sequence generated by this mutagenesis process on a pAB24F751-derived α-factor profibrolase fusion construct. This sequence, being thus derived, includes the amino acid changes of the corrected native sequence but lacks the 18 C-terminal residues of the Fib 51 profibrolase sequence. The segment coding for those C-terminal residues may be introduced if desired. Such constructs were made using the GAP promoter (pKS314 in the table) and the ADH2/GAP promoter (pKS313 in the table). A corresponding construct was made based on the K. lactis vector pAB38 (pKS362 in the table).

pKS314 and pKS362 were introduced into S. cerevisiae and K. lactis strains and the production and secretion of mature fibrolase from the transformants was analyzed by SDS gel electrophoresis and Western blot analysis. Both secreted mostly mature fibrolase.

pKS314 and pKS362 each contain two Lys-Arg processing sites, one at the α-factor-pro junction and another at the pro-mature junction. In order to minimize the possibility of there being insufficient KEX2 protease to process all of the profibrolase being produced, a synthetic oligonucleotide adapter was used to remove the Lys-Arg site at the α-factor-pro junction. These constructs are designated pKS316, pKS317, and pKS365.

The fibrolase products produced using the expression vectors listed in the above table were tested for fibrolase activity by either the azocasein hydrolysis assay or the fluorometric assay.

The azocasein hydrolysis assay is carried out as follows. A solution of 2.5 g azocasein in 50ml 1% NaHCO$_3$ is prepared with stirring and warming to 60° C. The resulting solution is then dialyzed overnight against 4 l of 1% NaHCO₃. A 50 μl portion of culture supernatent concentrated and dialyzed against 50 mM HEPES/pH 7.5 by ultrafiltration is then added to 1 ml of azocasein solution and the mixture is incubated for approximately 30 minutes-24 hours. One ml of 1.16 M perchloric acid is then added, the mixture is centrifuged for 10 minutes, and the absorbance of the supernatant is read at 390-440 nm against an appropriate control.

For the fluorometric assay, a fluorescein isothiocyanate casein (FITC-casein) assay reagent is prepared by mixing 1 part FITC-casein (5 mg/ml), 1 part 2X buffer (1X buffer = 50 mM HEPES-Na⁺, 150 mM NaCl, 0.5 mM ZnCl₂, pH 7.5). Recombinant fibrolase product and snake venom fibrolase standards are diluted in 1X buffer containing 100 μg/ml BSA as a stabilizer.

Assay reagent, 80 μl, is brought to 37° and 20 μl of fibrolase sample or standard is added. After a 90 minutes incubation at 37°, the reaction is stopped by the addition of 300 μl of 7% TCA. After 30-60 minutes at 4°, the mixture is centrifuged for 6 minutes at 12,000 rpm. A 200 μl portion of the supernatant is removed and added to 1 ml of 0.5 M Tris-HCl, pH 8.0 and the fluorescence measured at an excitation wavelength of 490 nm and an emission wavelength of 525 nm, and the value compared to the fibrolase standards. For determination of non-fibrolase protolytic activity, assays were done in the presence of 10 mM EDTA, an inhibitor of fibrolase.

All of the recombinant fibrolase products produced using the expression vectors listed in the table exhibited fibrolase activity in these assays.

Modifications of the above described modes for carrying out the invention that are obvious to those of skill in the fields of enzyme chemistry, genetic engineering and/or medicine are intended to be within the scope of the following claims.


## Claims

1. Recombinant DNA encoding a Crotalidus fibrolase.

2. The recombinant DNA of claim 1 wherein the fibrolase is southern copperhead fibrolase.

3. The recombinant DNA of claim 2 wherein the fibrolase comprises at least (a) amino acids 192-393 of the Fib 48 sequence shown in Figure 4, (b) amino acids 186-387 of the Fib 51 sequence of Figure 4 or (c) amino acids 185-387 of the Fib 51 sequence of Figure 4 with a Pro to Ser substitution at amino acid 307 of the Fib 51 sequence of Figure 4 and a Val to Met substitution at amino acid 354 of the Fib 51 sequence of Figure 4.

4. The recombinant DNA of claim 2 wherein the fibrolase is an analog of the fibrolase comprising at least (a) amino acids 192-393 of the Fib 48 sequence shown in Figure 4 (b) amino acids 186-387 of the Fib 51 sequence of Figure 4 or (c) amino acids 185-387 of the Fib 51 sequence of Figure 4 with a Pro to Ser substitution at amino acid 307 of the Fib 51 sequence of Figure 4 and a Val to Met substitution at amino acid 354 of the Fib 51 sequence of Figure 4.

5. An expression vector for expressing a Crotalidus fibrolase comprising the DNA of claim 1 and expression control sequences that are operably linked to said DNA and effective in directing expression of said DNA.

6. An expression vector for expressing southern copperhead fibrolase comprising the DNA of claim 2, 3 or 4 and expression control sequences that are operably linked to said DNA and effective in directing secretory expression of said DNA.

7. The expression vector of claim 6 wherein the expression vector is a yeast expression vector.

8. The expression vector of claim 7 wherein the expression control sequences include a yeast α-factor leader sequence for directing secretion of the fibrolase.

9. The expression vector of claim 8 wherein the DNA encodes the profibrolase sequence shown in Figure 7 and in which there is a Lys-Arg processing site at the pro-mature protein sequence.

10. A recombinant microorganism or cell containing the expression vector of claim 5 and being capable of producing a Crotalidus fibrolase.

11. A recombinant microorganism or cell containing the expression vector of claim 6 and being capable of producing southern copperhead fibrolase.

12. A recombinant yeast containing the expression vector of claims 7, 8 or 9.

13. A method of producing a Crotalidus fibrolase comprising growing the microorganism or cell of claim 10 under conditions which permit the expression of the fibrolase.

14. A method of producing southern copperhead fibrolase comprising growing the recombinant yeast cell of claim 12.

15. Recombinant homogeneous Crotalidus fibrolase free of snake proteins with which it is associated in venom.

16. The recombinant Crotalidus fibrolase of claim 15 wherein the fibrolase is southern copperhead fibrolase.

17. The recombinant southern copperhead fibrolase of claim 16 comprising at least (a) amino acids 192-393 of the Fib 48 sequence shown in Figure 4, (b) amino acids 186-387 of the Fib 51 sequence of Figure 4 or (c) amino acids 185-387 of the Fib 51 sequence of Figure 4 with a Pro to Ser substitution at amino acid 307 of the Fib 51 sequence of Figure 4 and a Val to Met substitution at amino acid 354 of the Fib 51 sequence of Figure 4, or an analog thereof.

18. A pharmaceutical composition for treating a thromboembolic condition comprising the recombinant Crotalidus fibrolase of claim 15, 16 or 17 admixed with a pharmaceutically acceptable injectable carrier.

19. A method of treating a patient for a thromboembolic condition comprising administering a therapeutically effective amount of the recombinant Crotalidus fibrolase of claim 15, 16 or 17 to the patient.

FIB48 MAP

```
    |_|                         ||      ||_|            |
____|_|_____SPHI___BSTE2_____SSPI____
    SACI                        SPHI    NDEI
      PSTI                      HIND3    STUI

    |                      ||   ||      |_||         |_|
____|_____AFL2_PSTI____SPEI_____BALI_____
    SACI                  AVA3  PSTI    NDEI         ASU2
    NAEI                                SSPI
```

2   TCAGGTTGACTTGAAAGAAGGAAGAGATTGCCTGTCTTCCAGCCAAATCCAGCCTCCAAA
    AGTCCAACTGAACTTTCTTCCTTCTCTAACGGACAGAAGGTCGGTTTAGGTCGGAGGTTT

    MetIleGlnValLeuLeuValThrIleCysLeuThrAlaPheProTyrGlnGlySerSer
62  ATGATCCAGGTTCTCTTGGTGACTATATGCTTAACAGCTTTTCCTTATCAAGGGAGCTCT
    TACTAGGTCCAAGAGAACCACTGATATACGAATTGTCGAAAAGGAATAGTTCCCTCGAGA

115 SACI

    IleIleLeuGluSerGlyAsnValAsnAspTyrGluValValTyrProArgLysValThr
122 ATAATCCTGGAATCTGGGAACGTGAATGATTATGAAGTAGTGTATCCACGAAAAGTTACT
    TATTAGGACCTTAGACCCTTGCACTTACTAATACTTCATCACATAGGTGCTTTTCAATGA

180 PSTI

    AlaValProArgGlyAlaValGlnProLysTyrGluAspAlaMetGlnTyrGluLeuLys
182 GCAGTGCCCAGAGGAGCAGTTCAGCCAAAGTATGAAGATGCCATGCAATATGAATTGAAA
    CGTCACGGGTCTCCTCGTCAAGTCGGTTTCATACTTCTACGGTACGTTATACTTAACTTT

    ValAsnGlyGluProValValLeuHisLeuGluLysAsnLysGlyLeuPheSerGluAsp
242 GTGAATGGAGAGCCAGTGGTCCTTCACCTGGAAAAAAATAAAGGACTTTTTTCAGAAGAT
    CACTTACCTCTCGGTCACCAGGAAGTGGACCTTTTTTTATTTCCTGAAAAAAGTCTTCTA

    TyrSerGluThrHisTyrSerProAspGlyArgGluIleThrThrTyrProLeuValGlu
302 TACAGCGAGACTCATTATTCCCCTGATGGCAGAGAAATTACAACATACCCCCTGGTTGAG
    ATGTCGCTCTGAGTAATAAGGGGACTACCGTCTCTTTAATGTTGTATGGGGGACCAACTC

    AspHisCysTyrTyrHisGlyArgIleGluAsnAspAlaAspSerThrAlaSerIleSer
362 GATCACTGCTATTATCATGGACGCATCGAGAATGATGCTGACTCAACTGCAAGCATCAGT
    CTAGTGACGATAATAGTACCTGCGTAGCTCTTACTACGACTGAGTTGACGTTCGTAGTCA

    AlaCysAsnGlyLeuLysGlyHisPheLysLeuGlnGlyGluMetTyrLeuIleGluPro
422 GCATGCAACGGTTTGAAAGGACATTTCAAGCTTCAAGGGGAGATGTACCTTATTGAACCA
    CGTACGTTGCCAAACTTTCCTGTAAAGTTCGAAGTTCCCCTCTACATGGAATAACTTGGT

422 SPHI, 449 HIND3

    LeuGluLeuSerAspSerGluAlaHisAlaValTyrLysTyrGluAsnValGluLysGlu
482 TTGGAGCTTTCCGACAGTGAAGCCCATGCAGTCTACAAATATGAAAATGTAGAAAAAGAG
    AACCTCGAAAGGCTGTCACTTCGGGTACGTCAGATGTTTATACTTTTACATCTTTTTCTC

FIG. 1-1

```
     AspGluAlaProLysMetCysGlyValThrGlnAsnTrpGluSerTyrGluProIleLys
542  GATGAGGCCCCCAAAATGTGTGGGGTAACCCAGAATTGGGAATCATATGAGCCCATCAAA
     CTACTCCGGGGGTTTTACACACCCCATTGGGTCTTAACCCTTAGTATACTCGGGTAGTTT

565 BSTE2, 585 NDEI

     LysAlaPheGlnLeuAsnLeuThrProGluGlnGlnGlyPheProGlnArgTyrValGlu
602  AAGGCCTTTCAGTTAAATCTTACTCCTGAACAACAAGGATTCCCCCAAAGATACGTTGAG
     TTCCGGAAAGTCAATTTAGAATGAGGACTTGTTGTTCCTAAGGGGGTTTCTATGCAACTC

603 STUI

     LeuValIleValAlaAspHisArgMetTyrThrLysTyrAsnGlyAspSerAspLysIle
662  CTTGTCATAGTTGCGGATCACAGAATGTACACGAAATACAATGGTGATTCAGATAAGATA
     GAACAGTATCAACGCCTAGTGTCTTACATGTGCTTTATGTTACCACTAAGTCTATTCTAT

     ArgGlnTrpIleTyrArgMetValAsnThrIleAsnGluIleTyrArgProLeuAsnIle
722  AGACAATGGATATATCGAATGGTCAACACTATAAATGAGATTTACAGACCTTTGAATATT
     TCTGTTACCTATATAGCTTACCAGTTGTGATATTTACTCTAAATGTCTGGAAACTTATAA

776 SSPI

     GlnPheValLeuValGlyLeuAspIleTrpSerLysLysAspLeuSerThrValThrSer
782  CAATTCGTACTGGTTGGCCTAGACATTTGGTCCAAGAAAGATTTGAGTACCGTGACATCA
     GTTAAGCATGACCAACCGGATCTGTAAACCAGGTTCTTTCTAAACTCATGGCACTGTAGT

     ValSerHisAspThrLeuAlaSerPheGluAsnTrpArgGlnThrAspLeuLeuAsnArg
842  GTATCACATGATACTTTGGCCTCATTTGAAAACTGGAGACAGACAGATTTGCTGAATCGC
     CATAGTGTACTATGAAACCGGAGTAAACTTTTGACCTCTGTCTGTCTAAACGACTTAGCG

     LysSerHisAspAsnAlaGlnLeuLeuThrAlaIleValPheAspGluGlyIleIleGly
902  AAAAGTCATGATAATGCCCAGTTACTCACGGCCATTGTCTTCGATGAAGGAATTATAGGA
     TTTTCAGTACTATTACGGGTCAATGAGTGCCGGTAACAGAAGCTACTTCCTTAATATCCT

     ArgAlaProLeuAlaGlyMetCysAspProMetPheSerValGlyIleValGluAspHis
962  AGAGCTCCCCTAGCCGGCATGTGTGACCCGATGTTTTCTGTAGGAATTGTTGAGGATCAT
     TCTCGAGGGGATCGGCCGTACACACTGGGCTACAAAAGACATCCTTAACAACTCCTAGTA

963 SACI, 974 NAEI

     SerAlaIleAsnLeuLeuValAlaLeuThrMetAlaHisGluLeuGlyHisAsnLeuGly
1022 AGTGCAATAAATCTTTTGGTTGCACTTACAATGGCCCATGAGCTGGGTCATAATCTGGGC
     TCACGTTATTTAGAAAACCAACGTGAATGTTACCGGGTACTCGACCCAGTATTAGACCCG

     MetAspHisAspGlyAsnGlnCysHisCysGlyAlaAsnSerCysValMetAlaAspThr
1082 ATGGATCATGATGGAAATCAGTGTCATTGCGGTGCTAACTCGTGCGTTATGGCTGACACA
     TACCTAGTACTACCTTTAGTCACAGTAACGCCACGATTGAGCACGCAATACCGACTGTGT

     LeuSerAsnGlnProSerLysLeuPheSerAspCysSerLysLysTyrTyrGlnLysPhe
1142 CTAAGTAATCAACCTTCCAAACTATTCAGCGATTGTAGTAAGAAATACTATCAGAAGTTT
     GATTCATTAGTTGGAAGGTTTGATAAGTCGCTAACATCATTCTTTATGATAGTCTTCAAA

     LeuLysValLysAsnProGlnCysIleLeuAsnLysProLeuArgThrAspThrValSer
1202 CTTAAGGTTAAAAACCCACAATGCATTCTCAATAAACCCTTGAGAACAGATACTGTTTCA
     GAATTCCAATTTTTGGGTGTTACGTAAGAGTTATTTGGGAACTCTTGTCTATGACAAAGT

1202 AFL2, 1222 AVA3

     ThrProValSerGlyAsnGluLeuLeuGluAlaOP
1262 ACTCCAGTTTCTGGAAATGAACTTTTGGAGGCGTGAGAAGAATGTGACTGTGGCTCTCCT
     TGAGGTCAAAGACCTTTACTTGAAAACCTCCGCACTCTTCTTACACTGACACCGAGAGGA

1320 PSTI
```

## FIG. I-2

GCAGTCTGCAGCAACAGGCAGTGTGTTGATGTGACTACAGCCTAATAATCAACCTCTGGC
CGTCAGACGTCGTTGTCCGTCACACAACTACACTGATGTCGGATTATTAGTTGGAGACCG

1327 PSTI

TTCTCTCAGATTTGATCTTGGAGATCCTTCTTTCAGAAGGTTTGGCTTCCCTGTAGTCCA
AAGAGAGTCTAAACTAGAACCTCTAGGAAGAAAGTCTTCCAAACCGAAGGGACATCAGGT

AAGAGACCCATCTGCCTGCATCCTACTAGTAAATCACTCTTAGCTTTCATATGGAATCTA
TTCTCTGGGTAGACGGACGTAGGATGATCATTTAGTGAGAATCGAAAGTATACCTTAGAT

1466 SPEI, 1489 NDEI

ACTTCTGCAATATTTCTTCTCCATATTTAATCTGTTTACCTTTTGCTGTAATCAAACCTT
TGAAGACGTTATAAAGAAGAGGTATAAATTAGACAAATGGAAAACGACATTAGTTTGGAA

1510 SSPI

TTCCCACCACAAAGCTCTATGGGCATGTACAACACCAACGGCTTATCTGCTGTCAAGAAA
AAGGGTGGTGTTTCGAGATACCCGTACATGTTGTGGTTGCCGAATAGACGACAGTTCTTT

AAAAATGGCCATTTTACCGTTTGCCAAAGCACATTTAATGCAACAAGTTCTGCCTTTTGA
TTTTTACCGGTAAAATGGCAAACGGTTTCGTGTAAATTACGTTGTTCAAGACGGAAAACT

1627 BALI

GCTGGTGTATTCGAAGTGAATGTTTACTCTCCCAAAATTTCATGCTGGCTTTCACAAGAT
CGACCACATAAGCTTCACTTACAAATGAGAGGGTTTTAAAGTACGACCGAAAGTGTTCTA

1691 ASU2

GTAGCTGCTTCCGTCAATAAACTAACTATTCTCATTCAAAAAAAA
CATCGACGAAGGCAGTTATTTGATTGATAAGAGTAAGTTTTTTTT

# FIG. I-3

FIB51 MAP

```
      ValThrIleCysLeuAlaAlaPheProTyrGlnGlySerSerIleIleLeuGluSerGly
   2  GTAACTATATGCTTAGCAGCTTTTCCTTATCAAGGGAGCTCTATAATCCTGGAATCTGGG
      CATTGATATACGAATCGTCGAAAAGGAATAGTTCCCTCGAGATATTAGGACCTTAGACCC

  37 SACI,

      AsnValAsnAspTyrGluValValTyrProArgLysValThrProValProArgGlyAla
  62  AACGTTAATGATTATGAAGTAGTGTATCCACGAAAAGTCACTCCAGTGCCCAGAGGAGCA
      TTGCAATTACTAATACTTCATCACATAGGTGCTTTTCAGTGAGGTCACGGGTCTCCTCGT

      ValGlnProLysTyrGluAspAlaMetGlnTyrGluPheLysValAsnGlyGluProVal
 122  GTTCAGCCAAAGTATGAAGATGCCATGCAATATGAATTTAAAGTGAATGGAGAGCCAGTG
      CAAGTCGGTTTCATACTTCTACGGTACGTTATACTTAAATTTCACTTACCTCTCGGTCAC

 158 AHA3,

      ValLeuHisLeuGluLysAsnLysGlyLeuPheSerGluAspTyrSerGluThrHisTyr
 182  GTCCTTCACCTGGAAAAAAATAAAGGACTTTTTTTCAGAAGATTACAGCGAGACTCATTAT
      CAGGAAGTGGACCTTTTTTTATTTCCTGAAAAAAGTCTTCTAATGTCGCTCTGAGTAATA

      SerProAspGlyArgGluIleThrThrTyrProLeuValGluAspHisCysTyrTyrHis
 242  TCCCCTGATGGCAGAGAAATTACAACATACCCCCTGGTTGAGGATCACTGCTATTATCAT
      AGGGGACTACCGTCTCTTTAATGTTGTATGGGGGACCAACTCCTAGTGACGATAATAGTA

      GlyArgIleGluAsnAspAlaAspSerThrAlaSerIleSerAlaCysAsnGlyLeuLys
 302  GGACGCATCGAGAATGATGCTGACTCAACTGCAAGCATCAGTGCATGCAACGGTTTGAAA
      CCTGCGTAGCTCTTACTACGACTGAGTTGACGTTCGTAGTCACGTACGTTGCCAAACTTT

 344 SPHI,

      GlyHisPheLysLeuGlnGlyGluMetTyrLeuIleGluProLeuGluLeuSerAspSer
 362  GGACATTTCAAGCTTCAAGGGGAGATGTACCTTATTGAACCGTTGGAGCTTTCCGACAGT
      CCTGTAAAGTTCGAAGTTCCCCTCTACATGGAATAACTTGGCAACCTCGAAAGGCTGTCA

 371 HIND3,

      GluAlaHisAlaValTyrLysTyrGluAsnValGluLysGluAspGluAlaProLysMet
 422  GAAGCCCATGCAGTCTACAAATATGAAAATGTAGAAAAAGAGGATGAGGCCCCCAAAATG
      CTTCGGGTACGTCAGATGTTTATACTTTTACATCTTTTTCTCCTACTCCGGGGGTTTTAC

      CysGlyValThrGlnAsnTrpGluSerTyrGluProIleLysLysAlaPheGlnLeuAsn
 482  TGTGGGGTAACCCAGAATTGGGAATCATATGAGCCCATCAAAAAGGCCTTTCAGTTAAAT
      ACACCCCATTGGGTCTTAACCCTTAGTATACTCGGGTAGTTTTTCCGGAAAGTCAATTTA

 487 BSTE2, 507 NDEI, 525 STUI,
```

# FIG. 2-1

## FIG. 2-2

```
          LeuThrProGluGlnGlnArgPheProGlnArgTyrValGlnLeuValIleValAlaAsp
542       CTTACTCCTGAACAACAAAGGTTCCCCCAAAGATATGTTCAGCTTGTCATAGTTGCAGAT
          GAATGAGGACTTGTTGTTTCCAAGGGGGTTTCTATACAAGTCGAACAGTATCAACGTCTA

          HisArgMetTyrMetLysTyrAsnAsnAspSerAsnLeuIleArgGlnTrpValHisGln
602       CACAGAATGTACATGAAATACAATAATGATTCAAATTTGATAAGACAATGGGTACATCAA
          GTGTCTTACATGTACTTTATGTTATTACTAAGTTTAAACTATTCTGTTACCCATGTAGTT

          IleValAsnThrIleAsnGluIleTyrArgProLeuAsnIleGlnPheThrLeuValGly
662       ATTGTCAACACTATAAATGAGATTTACAGACCTTTGAATATTCAATTCACACTGGTTGGC
          TAACAGTTGTGATATTTACTCTAAATGTCTGGAAACTTATAAGTTAAGTGTGACCAACCG

698 SSPI,

          LeuGluIleTrpSerAsnGlnAspLeuIleThrValThrSerValSerHisAspThrLeu
722       CTAGAAATTTGGTCCAACCAAGATTTGATTACCGTGACATCAGTATCACATGATACTTTG
          GATCTTTAAACCAGGTTGGTTCTAAACTAATGGCACTGTAGTCATAGTGTACTATGAAAC

          AlaSerPheGlyAsnTrpArgGluThrAspLeuLeuArgArgGlnArgHisAspAsnAla
782       GCCTCATTTGGAAACTGGAGAGAGACAGACTTGCTAAGGCGCCAAAGACATGATAATGCC
          CGGAGTAAACCTTTGACCTCTCTCTGTCTGAACGATTCCGCGGTTTCTGTACTATTACGG

819 NARI,

          GlnLeuLeuThrAlaIleAspPheAspGlyAspThrValGlyLeuAlaTyrValGlyGly
842       CAGTTACTCACGGCCATTGACTTTGATGGAGACACTGTAGGATTGGCTTATGTGGGCGGT
          GTCAATGAGTGCCGGTAACTGAAACTACCTCTGTGACATCCTAACCGAATACACCCGCCA

          MetCysGlnLeuLysHisProThrGlyValIleGlnAspHisSerAlaIleAsnLeuLeu
902       ATGTGCCAACTGAAGCATCCTACAGGAGTTATCCAGGATCATAGTGCAATAAATCTTTTG
          TACACGGTTGACTTCGTAGGATGTCCTCAATAGGTCCTAGTATCACGTTATTTAGAAAAC

          ValAlaLeuThrMetAlaHisGluLeuGlyHisAsnLeuGlyMetAsnHisAspGlyAsn
962       GTTGCACTTACAATGGCCCATGAGCTGGGTCATAATCTGGGCATGAATCATGATGGAAAT
          CAACGTGAATGTTACCGGGTACTCGACCCAGTATTAGACCCGTACTTAGTACTACCTTTA

          GlnCysHisCysGlyAlaAsnSerCysValMetAlaAlaValLeuSerAspGlnProSer
1022      CAGTGTCATTGCGGTGCTAACTCGTGCGTCATGGCTGCTGTGCTAAGTGATCAACCCTCC
          GTCACAGTAACGCCACGATTGAGCACGCAGTACCGACGACACGATTCACTAGTTGGGAGG

1069 BCLI,

          LysLeuPheSerAspCysSerLysLysAspTyrGlnThrPheLeuThrValAsnAsnPro
1082      AAACTATTCAGCGATTGTAGTAAGAAAGACTATCAGACGTTTCTTACGGTTAATAACCCA
          TTTGATAAGTCGCTAACATCATTCTTTCTGATAGTCTGCAAAGAATGCCAATTATTGGGT

          GlnCysIleLeuAsnLysProLeuArgThrAspThrValSerThrProValSerGlyAsn
1142      CAATGCATTCTCAATAAACCCTTGAGAACAGATACTGTTTCAACTCCAGTTTCTGGAAAT
          GTTACGTAAGAGTTATTTGGGAACTCTTGTCTATGACAAAGTTGAGGTCAAAGACCTTTA

1144 AVA3,

          GluLeuLeuGluAlaOP
1202      GAACTTTTGGAGGCGTGAGAAGAATGTGACTGTGGCTCTCCTGCAGTCTGCAGCAACAGG
          CTTGAAAACCTCCGCACTCTTCTTACACTGACACCGAGAGGACGTCAGACGTCGTTGTCC

1242 PSTI, 1249 PSTI,

1262      CAGTGTGTTGATGTGACTACAGCCTAATAATCAACCTCTGGCTTCTCTCAGATTTGATCT
          GTCACACAACTACACTGATGTCGGATTATTAGTTGGAGACCGAAGAGAGTCTAAACTAGA

1322      TGGAGATCCTTCTTTTCAGGAGGTTTGGCTTCCCTGTAGTCCAAAGAGACCCATCTGCCTG
          ACCTCTAGGAAGAAAGTCCTCCAAACCGAAGGGACATCAGGTTTCTCTGGGTAGACGGAC
```

1382 CATCCTACTAGTAAATCACTCTTAGCTTTCATATGGAATCTAACTTCTGCAATATTTCTT
GTAGGATGATCATTTAGTGAGAATCGAAAGTATACCTTAGATTGAAGACGTTATAAAGAA

1388 SPEI, 1411 NDEI, 1432 SSPI,

1442 CTCCATATTTAATCTGTAATCAAACCTTTTCCCACCACAAAGCTCTATGTGCATGTACAA
GAGGTATAAATTAGACATTAGTTTGGAAAAGGGTGGTGTTTCGAGATACACGTACATGTT

1502 CACCAACGGCTTATCTGCTGTCAAGAAAAAAAATGGCCATTTTACCGTTTGCCAAAGCAC
GTGGTTGCCGAATAGACGACAGTTCTTTTTTTTACCGGTAAAATGGCAAACGGTTTCGTG

1535 BALI,

1562 ATTTAATGCAACAAGTTCTGCCTTTTGAGCTGGTGTATTCGAAGTGAATGTTTACTCTCC
TAAATTACGTTGTTCAAGACGGAAAACTCGACCACATAAGCTTCACTTACAAATGAGAGG

1599 ASU2,

1622 CAAAATTTCATGCTGGCTTTCCAAGATGTAGCTGCTTCCGTCAATAAACTAACTATTCTC
GTTTTAAAGTACGACCGAAAGGTTCTACATCGACGAAGGCAGTTATTTGATTGATAAGAG

1682 ATTAAAAAAAAAAAAA
TAATTTTTTTTTTTTT

# FIG. 2-3

# FIG. 3-1

```
                70        80        90        100       110       120
Fib48  AATGATCCAGGTTCTCTTGGTGACTATATGCTTAACAGCTTTTCCTTATCAAGGGAGCTC
                                 *            *
Fib51                     GGTAAGTATATGCTTAGCAGCTTTTCCTTATCAAGGGAGCTC
                                  10        20        30        40

                130       140       150       160       170       180
Fib48  TATAATCCTGGAATCTGGGAACGTGAATGATTATGAAGTAGTGTATCCACGAAAAGTTAC
                                  *                            *
Fib51  TATAATCCTGGAATCTGGGAACGTTAATGATTATGAAGTAGTGTATCCACGAAAAGTCAC
          50        60        70        80        90        100 .

                190       200       210    .  220       230       240
Fib48  TGCAGTGCCCAGAGGAGCAGTTCAGCCAAAGTATGAAGATGCCATGCAATATGAATTGAA
         *                                                       *
Fib51  TCCAGTGCCCAGAGGAGCAGTTCAGCCAAAGTATGAAGATGCCATGCAATATGAATTTAA
          110       120       130       140       150       160

                250       260       270       280       290       300
Fib48  AGTGAATGGAGAGCCAGTGGTCCTTCACCTGGAAAAAAATAAAGGACTTTTTTCAGAAGA

Fib51  AGTGAATGGAGAGCCAGTGGTCCTTCACCTGGAAAAAAATAAAGGACTTTTTTCAGAAGA
          170       180       190       200       210       220

                310       320       330       340       350       360
Fib48  TTACAGCGAGACTCATTATTCCCCTGATGGCAGAGAAATTACAACATACCCCCTGGTTGA

Fib51  TTACAGCGAGACTCATTATTCCCCTGATGGCAGAGAAATTACAACATACCCCCTGGTTGA
          230       240       250       260       270       280

                370       380       390    .  400       410       420
Fib48  GGATCACTGCTATTATCATGGACGCATCGAGAATGATGCTGACTCAACTGCAAGCATCAG

Fib51  GGATCACTGCTATTATCATGGACGCATCGAGAATGATGCTGACTCAACTGCAAGCATCAG
          290       300       310       320       330       340

                430       440       450       460       470       480
Fib48  TGCATGCAACGGTTTGAAAGGACATTTCAAGCTTCAAGGGGAGATGTACCTTATTGAACC

Fib51  TGCATGCAACGGTTTGAAAGGACATTTCAAGCTTCAAGGGGAGATGTACCTTATTGAACC
          350       360       370       380       390       400

                490       500       510       520       530       540
Fib48  ATTGGAGCTTTCCGACAGTGAAGCCCATGCAGTCTACAAATATGAAAATGTAGAAAAAGA

Fib51  GTTGGAGCTTTCCGACAGTGAAGCCCATGCAGTCTACAAATATGAAAATGTAGAAAAAGA
          410       420       430       440       450       460

                550       560       570       580       590       600
Fib48  GGATGAGGCCCCCAAAATGTGTGGGGTAACCCAGAATTGGGAATCATATGAGCCCATCAA

Fib51  GGATGAGGCCCCCAAAATGTGTGGGGTAACCCAGAATTGGGAATCATATGAGCCCATCAA
          470       480       490       500       510       520

                610       620       630       640       650       660
Fib48  AAAGGCCTTTCAGTTAAATCTTACTCCTGAACAACAAGGATTCCCCCAAAGATACGTTGA
                                            * *                *     *
Fib51  AAAGGCCTTTCAGTTAAATCTTACTCCTGAACAACAAAGGTTCCCCCAAAGATATGTTCA
          530       540       550.      560       570       580

                670       680       690       700       710       720
Fib48  GCTTGTCATAGTTGCGGATCACAGAATGTACACGAAATACAATGGTGATTCAGATAAGAT
                        *                      *      **       *  **
Fib51  GCTTGTCATAGTTGCAGATCACAGAATGTACATGAAATACAATAATGATTCAAATTTGAT
          590       600       610       620       630       640
```

# FIG. 3-2

```
                730        740        750        760        770        780
Fib48  AAGACAATGGATATATCGAATGGTCAACACTATAAATGAGATTTACAGACCTTTGAATAT
                *     *     *      *
Fib51  AAGACAATGGGTACATCAAATTGTCAACACTATAAATGAGATTTACAGACCTTTGAATAT
                650        660        670        680        690        700


                790        800        810        820        830        840
Fib48  TCAATTCGTACTGGTTGGCCTAGACATTTGGTCCAAGAAAGATTTGAGTACCGTGACATC
                **                    *             **            *
Fib51  TCAATTCACACTGGTTGGCCTAGAAATTTGGTCCAACCAAGATTTGATTACCGTGACATC
                710        720        730        740        750        760


                850        860        870        880        890        900
Fib48  AGTATCACATGATACTTTGGCCTCATTTGAAAACTGGAGACAGACAGATTTGCTGAATCG
                                    *             *          *       *  **
Fib51  AGTATCACATGATACTTTGGCCTCATTTGGAAACTGGAGAGAGACAGACTTGCTAAGGCG
                770        780        790        800        810        820


                910 ·      920        930        940          950        960
Fib48  CAAAAGTCATGATAATGCCCAGTTACTCACGGCCATTGTCTTCGATGAAGGAATTATAGG
                *     *                           *     *     *  ** * *
Fib51  CCAAAGACATGATAATGCCCAGTTACTCACGGCCATTGACTTTGATGGAGACACTGTAGG
                830        840        850        860        870        880


                970        980        990        1000       1010       1020
Fib48  AAGAGCTCCCCTAGCCGGCATGTGTGACCCGATGTTTTCTGTAGGAATTGTTGAGGATCA
                ***    **** * *     *      ** * *   * ** *   **      *    * **
Fib51  ATTGGCTTATGTGGGCGGTATGTGCCAACTGAAGCATCCTACAGGAGTTATCCAGGATCA
                890        900        910        920        930        940


                1030       1040       1050       1060       1070       1080
Fib48  TAGTGCAATAAATCTTTTGGTTGCACTTACAATGGCCCATGAGCTGGGTCATAATCTGGG
Fib51  TAGTGCAATAAATCTTTTGGTTGCACTTACAATGGCCCATGAGCTGGGTCATAATCTGGG
                950        960        970        980        990        1000


                1090       1100       1110       1120       1130       1140
Fib48  CATGGATCATGATGGAAATCAGTGTCATTGCGGTGCTAACTCGTGCGTTATGGCTGACAC
                *                                         *        ****
Fib51  CATGAATCATGATGGAAATCAGTGTCATTGCGGTGCTAACTCGTGCGTCATGGCTGCTGT
                1010       1020       1030       1040       1050       1060


                1150       1160       1170       1180       1190       1200
Fib48  ACTAAGTAATCAACCTTCCAAACTATTCAGCGATTGTAGTAAGAAATACTATCAGAAGTT
                *     *        *                          *          *
Fib51  GCTAAGTGATCAACCCTCCAAACTATTCAGCGATTGTAGTAAGAAAGACTATCAGACGTT
                1070       1080       1090       1100       1110       1120


                1210       1220       1230       1240       1250       1260
Fib48  TCTTAAGGTTAAAAACCCACAATGCATTCTCAATAAACCCTTGAGAACAGATACTGTTTC
                *          *
Fib51  TCTTACGGTTAATAACCCACAATGCATTCTCAATAAACCCTTGAGAACAGATACTGTTTC
                1130       1140       1150       1160       1170       1180


                1270       1280       1290       1300       1310       1320
Fib48  AACTCCAGTTTCTGGAAATGAACTTTTGGAGGCGTGAGAAGAATGTGACTGTGGCTCTCC
Fib51  AACTCCAGTTTCTGGAAATGAACTTTTGGAGGCGTGAGAAGAATGTGACTGTGGCTCTCC
                1190       1200       1210       1220       1230       1240


                1330       1340       1350       1360       1370       1380
Fib48  TGCAGTCTGCAGCAACAGGCAGTGTGTTGATGTGACTACAGCCTAATAATCAACCTCTGG
Fib51  TGCAGTCTGCAGCAACAGGCAGTGTGTTGATGTGACTACAGCCTAATAATCAACCTCTGG
                1250       1260       1270       1280       1290       1300
```

```
             1390      1400      1410      1420      1430      1440
Fib48   CTTCTCTCAGATTTGATCTTGGAGATCCTTCTTTCAGAAGGTTTGGCTTCCCTGTAGTCC
                                                 *
Fib51   CTTCTCTCAGATTTGATCTTGGAGATCCTTCTTTCAGGAGGTTTGGCTTCCCTGTAGTCC
             1310      1320      1330      1340      1350      1360

             1450      1460      1470      1480      1490      1500
Fib48   AAAGAGACCCATCTGCCTGCATCCTACTAGTAAATCACTCTTAGCTTTCATATGGAATCT

Fib51   AAAGAGACCCATCTGCCTGCATCCTACTAGTAAATCACTCTTAGCTTTCATATGGAATCT
             1370      1380      1390      1400      1410      1420

             1510      1520      1530      1540      1550      1560
Fib48   AACTTCTGCAATATTTCTTCTCCATATTTAATCTGTTTACCTTTTGCTGTAATCAAACCT
                                                ***************
Fib51   AACTTCTGCAATATTTCTTCTCCATATTTAATC---------------TGTAATCAAACCT
             1430      1440      1450                      1460

             1570      1580      1590      1600      1610      1620
Fib48   TTTCCCACCACAAAGCTCTATGGGCATGTACAACACCAACGGCTTATCTGCTGTCAAGAA
                               *
Fib51   TTTCCCACCACAAAGCTCTATGTGCATGTACAACACCAACGGCTTATCTGCTGTCAAGAA
          1470      1480      1490      1500      1510      1520

             1630      1640      1650      1660      1670      1680
Fib48   AAAAAATGGCCATTTTACCGTTTGCCAAAGCACATTTAATGCAACAAGTTCTGCCTTTTG

Fib51   AAAAAATGGCCATTTTACCGTTTGCCAAAGCACATTTAATGCAACAAGTTCTGCCTTTTG
          1530      1540      1550      1560      1570      1580

             1690      1700      1710      1720      1730      1740
Fib48   AGCTGGTGTATTCGAAGTGAATGTTTACTCTCCCAAAATTTCATGCTGGCTTTCACAAGA
                                                              *
Fib51   AGCTGGTGTATTCGAAGTGAATGTTTACTCTCCCAAAATTTCATGCTGGCTTTC-CAAGA
          1590      1600      1610      1620      1630      1640

             1750      1760      1770      1780
Fib48   TGTAGCTGCTTCCGTCAATAAACTAACTATTCTCATTCAAAAAAAA
                                                 *
Fib51   TGTAGCTGCTTCCGTCAATAAACTAACTATTCTCATTAAAAAAAAAAAAA
          1650      1660      1670      1680      1690
```

94.3% identity in 1708 bp overlap

# FIG. 3-3

```
            10        20        30        40        50        60
Fib48   MIQVLLVTICLTAFPYQGSSIILESGNVNDYEVVYPRKVTAVPRGAVQPKYEDAMQYELK
            *                                  *                      *
Fib51      VTICLAAFPYQGSSIILESGNVNDYEVVYPRKVTPVPRGAVQPKYEDAMQYEFK
               10        20        30        40        50

            70        80        90       100       110       120
Fib48   VNGEPVVLHLEKNKGLFSEDYSETHYSPDGREITTYPLVEDHCYYHGRIENDADSTASIS

Fib51   VNGEPVVLHLEKNKGLFSEDYSETHYSPDGREITTYPLVEDHCYYHGRIENDADSTASIS
            60        70        80        90       100       110

           130       140       150       160       170       180
Fib48   ACNGLKGHFKLQGEMYLIEPLELSDSEAHAVYKYENVEKEDEAPKMCGVTQNWESYEPIK

Fib51   ACNGLKGHFKLQGEMYLIEPLELSDSEAHAVYKYENVEKEDEAPKMCGVTQNWESYEPIK
           120       130       140       150       160       170

           190       200       210       220       230       240
Fib48   KAFQLNLTPEQQGFPQRYVELVIVADHRMYTKYNGDSDKIRQWIYRMVNTINEIYRPLNI
               *         *         *       *   **    ****
Fib51   KAFQLNLTPEQQRFPQRYVQLVIVADHRMYMKYNNDSNLIRQWVHQIVNTINEIYRPLNI
           180       190       200       210       220       230

           250       260       270       280       290       300
Fib48   QFVLVGLDIWSKKDLSTVTSVSHDTLASFENWRQTDLLNRKSHDNAQLLTAIVFDEGIIG
            *    *   **   *            *     *      *  **         *    ****
Fib51   QFTLVGLEIWSNQDLITVTSVSHDTLASFGNWRETDLLRRQRHDNAQLLTAIDFDGDTVG
           240       250       260       270       280       290

           310       320       330       340       350       360
Fib48   RAPLAGMCDPMFSVGIVEDHSAINLLVALTMAHELGHNLGMDHDGNQCHCGANSCVMADT
            ***     ******  ***                           *             **
Fib51   LAYVGGMCQLKHPTGVIQDHSAINLLVALTMAHELGHNLGMNHDGNQCHCGANSCVMAAV
           300       310       320       330       340       350

           370       380       390       400       410
Fib48   LSNQPSKLFSDCSKKYYQKFLKVKNPQCILNKPLRTDTVSTPVSGNELLEA
            *              *   *   *  *
Fib51   LSDQPSKLFSDCSKKDYQTFLTVNNPQCILNKPLRTDTVSTPVSGNELLEA
           360       370       380       390       400
```

87.9 % identity in 405 residue overlap

# FIG. 4

FIBROLASE

StuI          PstI

|———— 717bp ————|

FIG. 5-1

ligate synthetic adapters
HindIII-StuI and PstI-SalI

                    MET ALA PHE GLN

AGCTTACAAAACAAA ATG GCC TTT CAG        CTGCAGAGATTTCGG
ATGTTTTGTTT     TAC CGG AAA GTC······GACGTCTCTAAAGCCAGCT
HindIII               StuI              PstI           SalI

ligate gene which encodes 231/aa
to promoter and to terminator
in pBR322 Δ RI Sal at BamHI site

ADH₂·GAPDH promotor          FIBROLASE
BamHI          HindIII   HindIII          SalI
|——— 1341 bp ———|       |——— 742 bp ———|

                                        SalI
                                              BamHI

                            BamHI     pPGAP-1
                                      BamHI (partial)
                                      SalI phosphatased

                     ADH₂·GAP promoter

          pBRF248        Fibrolase
          or pBRF251     F248 derived from SVG 48
                         F251 derived from SVG 51

          pBR322 Δ RI Sal    GAPDH terminator

FIG. 5-2

gel isolate BamHI expression
cassette and ligate into pAB24

BamHI     ADH$_2$· GAPDH promoter

HindIII

fibrolase gene

SalI

GAPDH
terminator

BamHI

ura 3

2-micron (B)

pAB24
F251
or
F248

pBR322

amp

ku 2d

2-micron (B)

GATCCTTCAATATGCGCACATACGCTGTTATGTTCAAGGTCCCTTCGTTTAAGAACGAAA
CTAGGAAGTTATACGCGTGTATGCGACAATACAAGTTCCAGGGAAGCAAATTCTTGCTTT

13 FSPI MSTI,

GCGGTCTTCCTTTTGAGGGATGTTTCAAGTTGTTCAAATCTATCAAATTTGCAAATCCCC
CGCCAGAAGGAAAACTCCCTACAAAGTTCAACAAGTTTAGATAGTTTAAACGTTTAGGGG

AGTCTGTATCTAGAGCGTTGAATCGGTGATGCGATTTGTTAATTAAATTGATGGTGTCAC
TCAGACATAGATCTCGCAACTTAGCCACTACGCTAAACAATTAATTTAACTACCACAGTG

129 XBAI,

CATTACCAGGTCTAGATATACCAATGGCAAACTGAGCACAACAATACCAGTCCGGATCAA
GTAATGGTCCAGATCTATATGGTTACCGTTTGACTCGTGTTGTTATGGTCAGGCCTAGTT

191 XBAI,

CTGGCACCATCTCTCCCGTAGTCTCATCTAATTTTTCTTCCGGATGAGGTTCCAGATATA
GACCGTGGTAGAGAGGGCATCAGAGTAGATTAAAAAGAAGGCCTACTCCAAGGTCTATAT

CCGCAACACCTTTATTATGGTTTCCCTGAGGGAATAATAGAATGTCCCATTCGAAATCAC
GGCGTTGTGGAAATAATACCAAAGGGACTCCCTTATTATCTTACAGGGTAAGCTTTAGTG

325 MST2, 350 ASU2,

CAATTCTAAACCTGGGCGAATTGTATTTCGGGTTTGTTAACTCGTTCCAGTCAGGAATGT
GTTAAGATTTGGACCCGCTTAACATAAAGCCCAAACAATTGAGCAAGGTCAGTCCTTACA

396 HPAI,

TCCACGTGAAGCTATCTTCCAGCAAAGTCTCCACTTCTTCATCAAATTGTGGAGAATACT
AGGTGCACTTCGATAGAAGGTCGTTTCAGAGGTGAAGAAGTAGTTTAACACCTCTTATGA

CCCAATGCTCTTATCTATGGGACTTCCGGGAAACACAGTACCGATACTTCCCAATTCGTC
GGGTTACGAGAATAGATACCCTGAAGGCCCTTTGTGTCATGGCTATGAAGGGTTAAGCAG

TTCAGAGCTCATTGTTTGTTTGAAGAGACTAATCAAAGAATCGTTTTCTCAAAAAAATTA
AAGTCTCGAGTAACAAACAAACTTCTCTGATTAGTTTCTTAGCAAAAGAGTTTTTTTAAT

545 SACI,

ATATCTTAACTGATAGTTTGATCAAAGGGGGCAAAACGTAGGGGCAAACAAACGGAAAAAT
TATAGAATTGACTATCAAACTAGTTTCCCCGTTTTGCATCCCCGTTTGTTTGCCTTTTTA

619 BCLI,

CGTTTCTCAAATTTTCTGATGCCAAGAACTCTAACCAGTCTTATCTAAAAAATTGCCTTAT

# FIG. 6-1

GCAAAGAGTTTAAAAGACTACGGTTCTTGAGATTGGTCAGAATAGATTTTTAACGGAATA

721 GATCCGTCTCTCCGGTTACAGCCTGTGTAACTGATTAATCCTGCCTTTCTAATCACCATT
CTAGGCAGAGAGGCCAATGTCGGACACATTGACTAATTAGGACGGAAAGATTAGTGGTAA

781 CTAATGTTTTAATTAAGGGATTTTGTCTTCATTAACGGCTTTCGCTCATAAAAATGTTAT
GATTACAAAATTAATTCCCTAAAACAGAAGTAATTGCCGAAAGCGAGTATTTTTACAATA

841 GACGTTTTGCCCGCAGGCGGGAAACCATCCACTTCACGAGACTGATCTCCTCTGCCGGAA
CTGCAAAACGGGCGTCCGCCCTTTGGTAGGTGAAGTGCTCTGACTAGAGGAGACGGCCTT

901 CACCGGGCATCTCCAACTTATAAGTTGGAGAAATAAGAGAATTTCAGATTGAGAGAATGA
GTGGCCCGTAGAGGTTGAATATTCAACCTCTTTATTCTCTTAAAGTCTAACTCTCTTACT

961 AAAAAAAAAAACCCTTAGTTCATAGGTCCATTCTCTTAGCGCAACTACAGAGAACAGGGGC
TTTTTTTTTTGGGAATCAAGTATCCAGGTAAGAGAATCGCGTTGATGTCTCTTGTCCCCG

1021 ACAAACAGGCAAAAAACGGGCACAACCTCAATGGAGTGATGCAACCTGCCTGGAGTAAAT
TGTTTGTCCGTTTTTTGCCCGTGTTGGAGTTACCTCACTACGTTGGACGGACCTCATTTA

1081 GATGACACAAGGCAATTGACCCACGCATGTATCTATCTCATTTTCTTACACCTTCTATTA
CTACTGTGTTCCGTTAACTGGGTGCGTACATAGATAGAGTAAAAGAATGTGGAAGATAAT

1141 CCTTCTGCTCTCTCTGATTTGGAAAAAGCTGAAAAAAAAGGTTGAAACCAGTTCCCTGAA
GGAAGACGAGAGAGACTAAACCTTTTTCGACTTTTTTTTCCAACTTTGGTCAAGGGACTT

1198 XMNI,

1201 ATTATTCCCCTACTTGACTAATAAGTATATAAAGACGGTAGGTATTGATTGTAATTCTGT
TAATAAGGGGATGAACTGATTATTCATATATTTCTGCCATCCATAACTAACATTAAGACA

1261 AAATCTATTTCTTAAACTTCTTAAATTCTACTTTTATAGTTAGTCTTTTTTTTAGTTTTA
TTTAGATAAAGAATTTGAAGAATTTAAGATGAAAATATCAATCAGAAAAAAAATCAAAAT

1317 AHA3,

1321 AAACACCAAGAACTTAGTTTCGAATAAACACACATAAACAAACAAGCTT
TTTGTGGTTCTTGAATCAAAGCTTATTTGTGTGTATTTGTTTGTTCGAA

1339 ASU2, 1364 HIND3,

# FIG. 6-2

# FIG. 7-1  INSERTION OF A KEX2 PROCESSING SITE INTO α-FACTOR/PROFIBROLASE

```
1                                       10                                      20          ***                 30
Met Arg Phe Pro Ser Ile Phe Thr Ala Val Leu Phe Ala Ala Ser Ser Ala Leu Ala Ala Pro Val Asn Thr Thr Thr Glu Asp Glu Thr
ATG AGA TTT CCT TCA ATT TTT ACT GCA GTT TTA TTC GCA GCA TCC TCC GCA TTA GCT GCT CCA GTC AAC ACT ACA ACA GAA GAT GAA ACG

                                        40                                      50              ***             60
Ala Gln Ile Pro Ala Glu Ala Val Ile Gly Tyr Leu Asp Leu Glu Gly Asp Phe Asp Val Ala Val Leu Pro Phe Ser Asn Ser Thr Asn
GCA CAA ATT CCG GCT GAA GCT GTC ATC GGT TAC TTA GAT TTA GAA GGG GAT TTC GAT GTT GCT GTT TTG CCA TTT TCC AAC AGC ACA AAT

                        ***             70                                      80                              90
Asn Gly Leu Leu Phe Ile Asn Thr Thr Ile Ala Ser Ile Ala Ala Lys Glu Glu Gly Val Ser Leu Asp Lys Arg Ser Ser Ile Ile Leu
AAC GGG TTA TTG TTT ATA AAT ACT ACT ATT GCC AGC ATT GCT GCT AAA GAA GAA GGG GTA TCT CTA GAT AAA AGG AGC TCT ATA ATC CTG

                                        100                                     110                             120
Glu Ser Gly Asn Val Asn Asp Tyr Glu Val Val Tyr Pro Arg Lys Val Thr Pro Val Pro Arg Gly Ala Val Gln Pro Lys Tyr Glu Asp
GAA TCT GGG AAC GTT AAT GAT TAT GAA GTA GTG TAT CCA CGA AAA GTC ACT CCA GTG CCC AGA GGA GCA GTT CAG CCA AAG TAT GAA GAT

                                        130                                     140                             150
Ala Met Gln Tyr Glu Phe Lys Val Asn Gly Glu Pro Val Val Leu His Leu Glu Lys Asn Lys Gly Leu Phe Ser Glu Asp Tyr Ser Glu
GCC ATG CAA TAT GAA TTT AAA GTG AAT GGA GAG CCA GTG GTC CTT CAC CTG GAA AAA AAT AAA GGA CTT TTT TCA GAA GAT TAC AGC GAG

                                        160                                     170                             180
Thr His Tyr Ser Pro Asp Gly Arg Glu Ile Thr Thr Tyr Pro Leu Val Glu Asp His Cys Tyr Tyr His Gly Arg Ile Glu Asn Asp Ala
ACT CAT TAT TCC CCT GAT GGC AGA GAA ATT ACA ACA TAC CCC CTG GTT GAG GAT CAC TGC TAT TAT CAT GGA CGC ATC GAG AAT GAT GCT

                                        190                                     200                             210
Asp Ser Thr Ala Ser Ile Ser Ala Cys Asn Gly Leu Lys Gly His Phe Lys Leu Gln Gly Glu Met Tyr Leu Ile Glu Pro Leu Glu Leu
GAC TCA ACT GCA AGC ATC AGT GCA TGC AAC GGT TTG AAA GGA CAT TTC AAG CTT CAA GGG GAG ATG TAC CTT ATT GAA CCG TTG GAG CTT

                                        220                                     230                             240
Ser Asp Ser Glu Ala His Ala Val Tyr Lys Tyr Glu Asn Val Glu Lys Glu Asp Glu Ala Pro Lys Met Cys Gly Val Thr Gln Asn Trp
TCC GAC AGT GAA GCC CAT GCA GTC TAC AAA TAT GAA AAT GTA GAA AAA GAG GAT GAG GCC CCC AAA ATG TGT GGG GTA ACC CAG AAT TGG
```

EP 0 323 722 A1

# FIG. 7-2

```
                                                        LYS ARG
                250              ***              ┌────────┐      260                              270
Glu Ser Tyr Glu Pro Ile Lys Lys Ala Phe Gln Leu Asn Leu Thr│Pro Glu│Gln Gln Arg Phe Pro Gln Arg Tyr Val Gln Leu Val Ile
GAA TCA TAT GAG CCC ATC AAA AAG GCC TTT CAG TTA AAT CTT ACT│CCT GAA│CAA CAA AGG TTC CCC CAA AGA TAT GTT CAG CTT GTC ATA
                                                        └────────┘

                                         Gly
                Asn Thr     280          ***         Asp Lys          290                              300
Val Ala Asp His Arg Met Tyr Met Lys Tyr Asn Asn Asp Ser Asn Leu Ile Arg Gln Trp Val His Gln Ile Val Asn Thr Ile Asn Glu
GTT GCA GAT CAC AGA ATG TAC ATG AAA TAC AAT AAT GAT TCA AAT TTG ATA AGA CAA TGG GTA CAT CAA ATT GTC AAC ACT ATA AAT GAG

                                 310                                  320                              330
Ile Tyr Arg Pro Leu Asn Ile Gln Phe Thr Leu Val Gly Leu Glu Ile Trp Ser Asn Gln Asp Leu Ile Thr Val Thr Ser Val Ser His
ATT TAC AGA CCT TTG AAT ATT CAA TTC ACA CTG GTT GGC CTA GAA ATT TGG TCC AAC CAA GAT TTG ATT ACC GTG ACA TCA GTA TCA CAT

                                 340                                  350                              360
Asp Thr Leu Ala Ser Phe Gly Asn Trp Arg Glu Thr Asp Leu Leu Arg Arg Gln Arg His Asp Asn Ala Gln Leu Leu Thr Ala Ile Asp
GAT ACT TTG GCC TCA TTT GGA AAC TGG AGA GAG ACA GAC TTG CTA AGG CGC CAA AGA CAT GAT AAT GCC CAG TTA CTC ACG GCC ATT GAC

                                 370                                  Ser                             390
Phe Asp Gly Asp Thr Val Gly Leu Ala Tyr Val Gly Gly Met Cys Gln Leu Lys His Pro Thr Gly Val Ile Gln Asp His Ser Ala Ile
TTT GAT GGA GAC ACT GTA GGA TTG GCT TAT GTG GGC GGT ATG TGC CAA CTG AAG CAT CCT ACA GGA GTT ATC CAG GAT CAT AGT GCA ATA

                                 400                                  410                             420
Asn Leu Leu Val Ala Leu Thr Met Ala His Glu Leu Gly His Asn Leu Gly Met Asn His Asp Gly Asn Gln Cys His Cys Gly Ala Asn
AAT CTT TTG GTT GCA CTT ACA ATG GCC CAT GAG CTG GGT CAT AAT CTG GGC ATG AAT CAT GAT GGA AAT CAG TGT CAT TGC GGT GCT AAC

                Met          430                                      440                             450
Ser Cys Val Met Ala Ala Val Leu Ser Asp Gln Pro Ser Lys Leu Phe Ser Asp Cys Ser Lys Lys Asp Tyr Gln Thr Phe Leu Thr Val
TCG TGC GTC ATG GCT GCT GTG CTA AGT GAT CAA CCC TCC AAA CTA TTC AGC GAT TGT AGT AAG AAA GAC TAT CAG ACG TTT CTT ACG GTT

                                 460                                  470                      478
Asn Asn Pro Gln Cys Ile Leu Asn Lys Pro Leu Arg Thr Asp Thr Val Ser Thr Pro Val Ser Gly Asn Glu Leu Leu Glu Ala OP
AAT AAC CCA CAA TGC ATT CTC AAT AAA CCC TTG AGA ACA GAT ACT GTT TCA ACT CCA GTT TCT GGA AAT GAA CTT TTG GAG GCG TGA
```

EP 0 323 722 A1

# FIG. 8-1

## pKS308

EP 0 323 722 A1

```
1    GGATCCCCAGCTTAGTTCATAGGTCCATTCTCTTAGCGCAACTACAGAGAACAGGGGCACAAACAGGCAAAAAACGGGCACAACCTCAATGGAGTGATGCAACCTGCCTGGAGTAAATGATG
     CCTAGGGGTCGAATCAAGTATCCAGGTAAGAGAATCGCGTTGATGTCTCTTGTCCCCGTGTTTGTCCGTTTTTTGCCCGTGTTGGAGTTACCTCACTACGTTGGACGGACCTCATTTACTAC
     BamHI
```

```
123  ACACAAGGGAATTGACCCACGCATGTATCTATCTCATTTTCTTACACCTTCTATTACCTTCTGCTCTCTCTGATTTGGAAAAAGCTGAAAAAAAAGGTTGAAACCAGTTCCCTGAAATTA
     TGTGTTCCGTTAACTGGGTGCGTACATAGATAGAGTAAAAGAATGTGGAAGATAATGGAAGACGAGAGAGACTAAACCTTTTTCGACTTTTTTTTCCAACTTTGGTCAAGGGACTTTAAT
```

```
243  TTCCCCTACTTGACTAATAAGTATATAAAGACGGTAGGTATTGATTGTAATTCTGTAAATCTATTTCTTAAACTTCTTAAATTCTACTTTTATAGTTAGTCTTTTTTTTAGTTTTAAAAC
     AAGGGGATGAACTGATTATTCATATATTTCTGCCATCCATAACTAACATTAAGACATTTAGATAAAGAATTTGAAGAATTTAAGATGAAAATATCAATCAGAAAAAAAATCAAAATTTTG
```

```
                                   MetArgPheProSerIlePheThrAlaValLeuPheAlaAlaSerSerAlaLeuAlaAlaProValAsnThrThrThr
363  ACCAAGAACTTAGTTTCGAATAAACACACATAAACAAACACCATGAGATTTCCTTCAATTTTTACTGCAGTTTTATTCGCAGCATCCTCCGCATTAGCTGCTCCAGTCAACACTACAACA
     TGGTTCTTGAATCAAAGCTTATTTGTGTGTATTTGTTTGTGGTACTCTAAAGGAAGTTAAAAATGACGTCAAAATAAGCGTCGTAGGAGGCGTAATCGACGAGGTCAGTTGTGATGTTGT
                                                                    PstI
```

```
     GluAspGluThrAlaGlnIleProAlaGluAlaValIleGlyTyrLeuAspLeuGluGlyAspPheAspValAlaValLeuProPheSerAsnSerThrAsnAsnGlyLeuLeuPheIle
483  GAAGATGAAACGGCACAAATTCCGGCTGAAGCTGTCATCGGTTACTTAGATTTAGAAGGGGATTTCGATGTTGCTGTTTTGCCATTTTCCAACAGCACAAATAACGGGGTTATTGTTTATA
     CTTCTACTTTGCCGTGTTTAAGGCCGACTTCGACAGTAGCCAATGAATCTAAATCTTCCCCTAAAGCTACAACGACAAAACGGTAAAAGGTTGTCGTGTTTATTGCCCAATAACAAATAT
```

```
     AsnThrThrIleAlaSerIleAlaAlaLysGluGluGlyValSerLeuAspLysArgGlnGlnArgPheProGlnArgTyrValGlnLeuValIleValAlaAspHisArgMetAsnThr
603  AATACTACTATTGCCAGCATTGCTGCTAAAGAAGAAGGGGTATCTCTAGATAAAAGACAACAAAGATTCCCCCAAAGATACGTTCAGCTGGTCATAGTTGCGGATCACAGAATGAATACG
     TTATGATGATAACGGTCGTAACGACGATTTCTTCTTCCCCATAGAGATCTATTTTCTGTTGTTTCTAAGGGGGTTTCTATGCAAGTCGACCAGTATCAACGCCTAGTGTCTTACTTATGC
                                                   XbaI                                              PvuII
```

# FIG. 8-2

```
      LysTyrAsnGlyAspSerAspLysIleArgGlnTrpValHisGlnIleValAsnThrIleAsnGluIleTyrArgProLeuAsnIleGlnPheThrLeuValGlyLeuGluIleTrpSer
 723  AAATACAATGGTGATTCAGATAAGATAAGACAATGGGTTCACCAGATTGTCAACACTATAAATGAGATTTACAGACCTTTGAATATTCAATTCACACTGGTTGGCCTAGAAATTTGGTCC
      TTTATGTTACCACTAAGTCTATTCTATTCTGTTACCCAAGTGGTCTAACAGTTGTGATATTTACTCTAAATGTCTGGAAACTTATAAGTTAAGTGTGACCAACCGGATCTTTAAACCAGG

      AsnGlnAspLeuIleThrValThrSerValSerHisAspThrLeuAlaSerPheGlyAsnTrpArgGluThrAspLeuLeuArgArgGlnArgHisAspAsnAlaGlnLeuLeuThrAla
 843  AACCAAGATTTGATTACCGTGACATCAGTATCACATGATACTTTGGCCTCATTTGGAAACTGGAGAGAGACAGACTTGCTAAGGCGCCAAAGACATGATAATGCCCAGTTACTCACGGCC
      TTGGTTCTAAACTAATGGCACTGTAGTCATAGTGTACTATGAAACCGGAGTAAACCTTTGACCTCTCTCTGTCTGAACGATTCCGCGGTTTCTGTACTATTACGGGTCAATGAGTGCCGG

      IleAspPheAspGlyAspThrValGlyLeuAlaTyrValGlyGlyMetCysGlnLeuLysHisSerThrGlyValIleGlnAspHisSerAlaIleAsnLeuLeuValAlaLeuThrMet
 963  ATTGACTTTGATGGAGACACTGTAGGATTGGCTTATGTGGGCGGTATGTGCCAACTGAAGCACTCCACTGGAGTTATCCAGGATCATAGTGCAATAAATCTTTTGGTTGCACTTACAATG
      TAACTGAAACTACCTCTGTGACATCCTAACCGAATACACCCGCCATACACGGTTGACTTCGTGAGGTGACCTCAATAGGTCCTAGTATCACGTTATTTAGAAAACCAACGTGAATGTTAC

      AlaHisGluLeuGlyHisAsnLeuGlyMetAsnHisAspGlyAsnGlnCysHisCysGlyAlaAsnSerCysValMetAlaAlaMetLeuSerAspGlnProSerLysLeuPheSerAsp
1083  GCCCATGAGCTGGGTCATAATCTGGGCATGAATCATGATGGAAATCAGTGTCATTGCGGTGCTAACTCGTGCGTCATGGCTGCCATGTTAAGTGATCAACCCTCCAAACTATTCAGCGAT
      CGGGTACTCGACCCAGTATTAGACCCGTACTTAGTACTACCTTTAGTCACAGTAACGCCACGATTGAGCACGCAGTACCGACGGTACAATTCACTAGTTGGGAGGTTTGATAAGTCGCTA

      CysSerLysLysAspTyrGlnThrPheLeuThrValAsnAsnProGlnCysIleLeuAsnLysProAM  OC
1203  TGTAGTAAGAAAGACTATCAGACGTTTCTTACGGTTAATAACCCACAATGCATTCTCAATAAACCCAGTAAGTCGACTTTGTTCCCACTGTACTTTTAGCTCGTACAAAATACAATATA
      ACATCATTCTTTCTGATAGTCTGCAAAGAATGCCAATTATTGGGTGTTACGTAAGAGTTATTTGGGATCATTCAGCTGAAACAAGGGTGACATGAAAATCGAGCATGTTTTATGTTATAT
                                                       Nsil                              SalI

1323  CTTTTCATTTCTCCGTAAACAACATGTTTTCCCATGTAATATCCTTTTCTATTTTTCGTTCCGTTACCAACTTTACACATACTTTATATAGCTATTCACTTCTATACACTAAAAAACTAA
      GAAAAGTAAAGAGGCATTTGTTGTACAAAAGGGTACATTATAGGAAAAGATAAAAAGCAAGGCAATGGTTGAAATGTGTATGAAATATATCGATAAGTGAAGATATGTGATTTTTTGATT

1443  GACAATTTTAATTTTGCTGCCTGCCATATTTCAATTTGTTATAAATTCCTATAATTTATCCTATTAGTAGCTAAAAAAAAGATGAATGTGAATCGAATCCTAAGAGAATTCGGATCC
      CTGTTAAAATTAAAACGACGGACGGTATAAAGTTAAACAATATTTAAGGATATTAAATAGGATAATCATCGATTTTTTTCTACTTACACTTAGCTTAGGATTCTCTTAAGCCTAGG
                                                                                                              EcoRI  BamHI
```

EP 0 323 722 A1

# FIG. 9-1

1 GGATCCCCAGCTTAGTTCATAGGTCCATTCTCTTAGCGCAACTACAGAGAACAGGGGCACAAACAGGCAAAAAACGGGCACAACCTCAATGGAGTGATGCAACCTGCCTGGAGTAAATGATG
CCTAGGGGTCGAATCAAGTATCCAGGTAAGAGAATCGCGTTGATGTCTCTTGTCCCCGTGTTTGTCCGTTTTTTGCCCGTGTTGGAGTTACCTCACTACGTTGGACGGACCTCATTTACTAC
BamHI

123 ACACAAGGCAATTGACCCACGCATGTATCTATCTCATTTTCTTACACCTTCTATTACCTTCTGCTCTCTCTGATTTGGAAAAAGCTGAAAAAAAAGGTTGAAACCAGTTCCCTGAAATTA
TGTGTTCCGTTAACTGGGTGCGTACATAGATAGAGTAAAAGAATGTGGAAGATAATGGAAGACGAGAGAGACTAAACCTTTTTCGACTTTTTTTTCCAACTTTGGTCAAGGGACTTTAAT

243 TTCCCCTACTTGACTAATAAGTATATAAAGACGGTAGGTATTGATTGTAATTCTGTAAATCTATTTCTTAAACTTCTTAAATTCTACTTTTATAGTTAGTCTTTTTTTTAGTTTTAAAAC
AAGGGGATGAACTGATTATTCATATATTTCTGCCATCCATAACTAACATTAAGACATTTAGATAAAGAATTTGAAGAATTTAAGATGAAAATATCAATCAGAAAAAAAATCAAAATTTTG

MetArgPheProSerIlePheThrAlaValLeuPheAlaAlaSerSerAlaLeuAlaAlaProValAsnThrThrThr
363 ACCAAGAACTTAGTTTCGAATAAACACACATAAACAAACACCATGAGATTTCCTTCAATTTTTACTGCAGTTTTATTCGCAGCATCCTCCGCATTAGCTGCTCCAGTCAACACTACAACA
TGGTTCTTGAATCAAAGCTTATTTGTGTGTATTTGTTTGTGGTACTCTAAAGGAAGTTAAAAATGACGTCAAAATAAGCGTCGTAGGAGGCGTAATCGACGAGGTCAGTTGTGATGTTGT
PstI

GluAspGluThrAlaGlnIleProAlaGluAlaValIleGlyTyrLeuAspLeuGluGlyAspPheAspValAlaValLeuProPheSerAsnSerThrAsnAsnGlyLeuLeuPheIle
483 GAAGATGAAACGGCACAAATTCCGGCTGAAGCTGTCATCGGTTACTTAGATTTAGAAGGGGATTTCGATGTTGCTGTTTTGCCATTTTCCAACAGCACAAATAACGGGTTATTGTTTATA
CTTCTACTTTGCCGTGTTTAAGGCCGACTTCGACAGTAGCCAATGAATCTAAATCTTCCCCTAAAGCTACAACGACAAAACGGTAAAAGGTTGTCGTGTTTATTGCCCAATAACAAATAT

AsnThrThrIleAlaSerIleAlaAlaLysGluGluGlyValSerLeuAspLysArgSerSerIleIleLeuGluSerGlyAsnValAsnAspTyrGluValValTyrProArgLysVal
603 AATACTACTATTGCCAGCATTGCTGCTAAAGAAGAAGGGGTATCTCTAGATAAAAGGAGCTCTATAATCCTGGAATCTGGGAACGTTAATGATTATGAAGTAGTGTATCCACGAAAAGTC
TTATGATGATAACGGTCGTAACGACGATTTCTTCTTCCCCATAGAGATCTATTTTCCTCGAGATATTAGGACCTTAGACCCTTGCAATTACTAATACTTCATCACATAGGTGCTTTTCAG
Xbal          Sacl

ThrProValProArgGlyAlaValGlnProLysTyrGluAspAlaMetGlnTyrGluPheLysValAsnGlyGluProValValLeuHisLeuGluLysAsnLysGlyLeuPheSerGlu
723 ACTCCAGTGCCCAGAGGAGCAGTTCAGCCAAAGTATGAAGATGCCATGCAATATGAATTTAAAGTGAATGGAGAGCCAGTGGTCCTTCACCTGGAAAAAAATAAAGGACTTTTTTCAGAA
TGAGGTCACGGGTCTCCTCGTCAAGTCGGTTTCATACTTCTACGGTACGTTATACTTAAATTTCACTTACCTCTCGGTCACCAGGAAGTGGACCTTTTTTTATTTCCTGAAAAAAGTCTT

# FIG. 9-2

AspTyrSerGluThrHisTyrSerProAspGlyArgGluIleThrThrTyrProLeuValGluAspHisCysTyrTyrHisGlyArgIleGluAsnAspAlaAspSerThrAlaSerIle
GATTACAGCGAGACTCATTATTCCCCTGATGGCAGAGAAATTACAACATACCCCCTGGTTGAGGATCACTGCTATTATCATGGACGCATCGAGAATGATGCTGACTCAACTGCAAGCATC
CTAATGTCGCTCTGAGTAATAAGGGGACTACCGTCTCTTTAATGTTGTATGGGGGACCAACTCCTAGTGACGATAATAGTACCTGCGTAGCTCTTACTACGACTGAGTTGACGTTCGTAG

SerAlaCysAsnGlyLeuLysGlyHisPheLysLeuGlnGlyGluMetTyrLeuIleGluProLeuGluLeuSerAspSerGluAlaHisAlaValTyrLysTyrGluAsnValGluLys
AGTGCATGCAACGGTTTGAAAGGACATTTCAAGCTTCAAGGGGAGATGTACCTTATTGAACCGTTGGAGCTTTCCGACAGTGAAGCCCATGCAGTCTACAAATATGAAAATGTAGAAAAA
TCACGTACGTTGCCAAACTTTCCTGTAAAG<u>TTCGAAG</u>TTCCCCTCTACATGGAATAACTTGGCAACCTCGAAAGGCTGTCACTTCGGGTACGTCAGATGTTTATACTTTTACATCTTTTT
                              HinDIII

GluAspGluAlaProLysMetCysGlyValThrGlnAsnTrpGluSerTyrGluProIleLysLysAlaPheGlnLeuAsnLeuThrProGluGlnGlnArgPheProGlnArgTyrVal
GAGGATGAGGCCCCCAAAATGTGTGGGGTAACCCAGAATTGGGAATCATATGAGCCCATCAAAAAGGCCTTTCAGTTAAATCTTACTCCTGAACAACAAAGGTTCCCCCAAAGATATGTT
CTCCTACTCCGGGGGGTT<u>TTACACACCCCATTGGG</u>TCTTAACCCTTAGTATACTCGGGTAGTTTTTCCGGAAAGTCAATTTAGAATGAGGACTTGTTGTTTCCAAGGGGGGTTTCTATACAA
                  BstEII

GlnLeuValIleValAlaAspHisArgMetTyrMetLysTyrAsnAsnAspSerAsnLeuIleArgGlnTrpValHisGlnIleValAsnThrIleAsnGluIleTyrArgProLeuAsn
CAGCTTGTCATAGTTGCAGATCACAGAATGTACATGAAATACAATAATGATTCAAATTTGATAAGACAATGGGTACATCAAATTGTCAACACTATAAATGAGATTTACAGACCTTTGAAT
GTCGAACAGTATCAACGTCTAGTGTCTTACATGTACTTTATGTTATTACTAAGTTTAAACTATTCTGTTACCCATGTAGTTTAACAGTTGTGATATTTACTCTAAATGTCTGGAAACTTA

IleGlnPheThrLeuValGlyLeuGluIleTrpSerAsnGlnAspLeuIleThrValThrSerValSerHisAspThrLeuAlaSerPheGlyAsnTrpArgGluThrAspLeuLeuArg
ATTCAATTCACACTGGTTGGCCTAGAAATTTGGTCCAACCAAGATTTGATTACCGTGACATCAGTATCACATGATACTTTGGCCTCATTTGGAAACTGGAGAGAGACAGACTTGCTAAGG
TAAGTTAAGTGTGACCAACCGGATCTTTAAACCAGGTTGGTTCTAAACTAATGGCACTGTAGTCATAGTGTACTATGAAACCGGAGTAAACCTTTGACCTCTCTCTGTCTGAACGATTCC

ArgGlnArgHisAspAsnAlaGlnLeuLeuThrAlaIleAspPheAspGlyAspThrValGlyLeuAlaTyrValGlyGlyMetCysGlnLeuLysHisProThrGlyValIleGlnAsp
CGCCAAAGACATGATAATGCCCAGTTACTCACGGCCATTGACTTTGATGGAGACACTGTAGGATTGGCTTATGTGGGCGGTATGTGCCAACTGAAGCATCCTACAGGAGTTATCCAGGAT
GCGGTTTCTGTACTATTACGGGTCAATGAGTGCCGGTAACTGAAACTACCTCTGTGACATCCTAACCGAATACACCCGCCATACACGGTTGACTTCGTAGGATGTCCTCAATAGGTCCTA

EP 0 323 722 A1

# FIG. 9-3

```
         HisSerAlaIleAsnLeuLeuValAlaLeuThrMetAlaHisGluLeuGlyHisAsnLeuGlyMetAsnHisAspGlyAsnGlnCysHisCysGlyAlaAsnSerCysValMetAlaAla
1563  CATAGTGCAATAAATCTTTTGGTTGCACTTACAATGGCCCATGAGCTGGGTCATAATCTGGGCATGAATCATGATGGAAATCAGTGTCATTGCGGTGCTAACTCGTGCGTCATGGCTGCT
      GTATCACGTTATTTAGAAAACCAACGTGAATGTTACCGGGTACTCGACCCAGTATTAGACCCGTACTTAGTACTACCTTTAGTCACAGTAACGCCACGATTGAGCACGCAGTACCGACGA


         ValLeuSerAspGlnProSerLysLeuPheSerAspCysSerLysLysAspTyrGlnThrPheLeuThrValAsnAsnProGlnCysIleLeuAsnLysProLeuArgThrAspThrVal
1683  GTGCTAAGTGATCAACCCTCCAAACTATTCAGCGATTGTAGTAAGAAAGACTATCAGACGTTTCTTACGGTTAATAACCCACAATGCATTCTCAATAAACCCCTTGAGAACAGATACTGTT
      CACGATTCACTAGTTGGGAGGTTTGATAAGTCGCTAACATCATTCTTTCTGATAGTCTGCAAAGAATGCCAATTATTGGGTGTTACGTAAGAGTTATTTGGGAACTCTTGTCTATGACAA
                                                                                                    NsiI

         SerThrProValSerGlyAsnGluLeuLeuGluAlaOP
1803  TCAACTCCAGTTTCTGGAAATGAACTTTTGGAGGCGTGAGAAGAATGTGACTGTGGCTCTCCTGCAGGTCGACTTGGTTGAACACGTTGCCAAGGCTTAAGTGAATTTACTTTAAAGTCT
      AGTTGAGGTCAAAGACCTTTACTTGAAAACCTCCGCACTCTTCTTACACTGACACCGAGAGGACGTCCAGCTGAACCAACTTGTGCAACGGTTCCGAATTCACTTAAATGAAATTTCAGA
                                                                                     PstI    SalI

1923  TGCATTTAAATAAATTTTCTTTTTATAGCTTTATGACTTAGTTTCAATTTATATACTATTTTAATGACATTTTCGATTCATTGATTGAAAGCTTTGTGTTTTTTCTTGATGCGCTATTGC
      ACGTAAATTTATTTAAAAGAAAAATATCGAAATACTGAATCAAAGTTAAATATATGATAAAATTACTGTAAAAGCTAAGTAACTAACTTTCGAAACACAAAAAAGAACTACGCGATAACG
                                                                                                        HinDIII

2043  ATTGTTCTTGTCTTTTTCGCCACATGTAATATCTGTAGTAGATACCTGATACATTGTGGATGCTGAGTGAAATTTTAGTTAATAATGGAGGCGCTCTTAATAATTTTGGGGATATTGGCT
      TAACAAGAACAGAAAAAGCGGTGTACATTATAGACATCATCTATGGACTATGTAACACCTACGACTCACTTTAAAATCAATTATTACCTCCGCGAGAATTATTAAAACCCCTATAACCGA

2163  TTTTTTTTTAAAGTTTACAAATGAATTTTTTCCGCCAGGATAACGATTCTGAAGTTACTCTTAGCGTTCCTATCGGTACAGCCATCAAATCATGCCTATAAATCATGCCTATATTTGCGT
      AAAAAAAAATTTCAAATGTTTACTTAAAAAAGGCGGTCCTATTGCTAAGACTTCAATGAGAATCGCAAGGATAGCCATGTCGGTAGTTTAGTACGGATATTTAGTACGGATATAAACGCA
```

EP 0 323 722 A1

2283 GCAGTCAGTATCATCTACATGAAAAAAAACTCCCGCAATTTCTTATAGAATACGTTGAAAATTAAATGTACGCGCCAAGATAAGATAACATATATCTAGCTAGATGCAGTAATATACACAG
CGTCAGTCATAGTAGATGTACTTTTTTTTGAGGGCGTTAAAGAATATCTTATGCAACTTTTAATTTACATGCGCGGTTCTATTCTATTGTATATAGATCGATCTACGTCATTATATGTGTC

2403 ATTCCCGCGGACGTGGGAAGGAAAAAATTAGATAACAAAATCTGAGTGATATGGAAATTCCGCTGTATAGCTCATATCTTTCCCTTCAACACCAGAAATGTAAAAATCTTGTTACGAAGG
TAAGGGCGCCTGCACCCTTCCTTTTTTAATCTATTGTTTTAGACTCACTATACCTTTAAGGCGACATATCGAGTATAGAAAGGGAAGTTGTGGTCTTTACATTTTTAGAACAATGCTTCC
     SacII

2523 ATCTTTTTGCTAATGTTTCTCGCTCAATCCTCATTTCTTCCCTACGAAGAGTCAAATCTACTTGTTTTCTGCCGGTATCAAGATCCATATCTTCTAGTTTCACCATCAAAGTCCAATTTC
TAGAAAAACGATTACAAAGAGCGAGTTAGGAGTAAAGAAGGGATGCTTCTCAGTTTAGATGAACAAAAGACGGCCATAGTTCTAGGTATAGAAGATCAAAGTGGTAGTTTCAGGTTAAAG

2643 TAGTATACAGTTTATGTCCCAACGTAACAGACAATCAAAATTGGAAAGGATAAGTATCCTTCAAAGAATGATTCTGCGCTGGCTCCTGAACCGCCTAATGGGAACAGAGAAGTCCAAAAC
ATCATATGTCAAATACAGGGGTTGCATTGTCTGTTAGTTTTAACCTTTCCTATTCATAGGAAGTTTCTTACTAAGACGCGACCGAGGACTTGGCGGATTACCCTTGTCTCTTCAGGTTTTG

2763 GATGCTATAAGAACCAGAAATAAAACGATAAAACCATACCAGGATCC
CTACGATATTCTTGGTCTTTATTTTGCTATTTTGGTATGGTCCTAGG
                                          BamHI

# FIG. 9-4
pKS 311

EP 0 323 722 A1

# FIG. 10-1

1 GGATCCCCAGCTTAGTTCATAGGTCCATTCTCTTAGCGCAACTACAGAGAACAGGGGCACAAACAGGCAAAAAACGGGCACAACCTCAATGGAGTGATGCAACCTGCCTGGAGTAAATGATG
CCTAGGGGTCGAATCAAGTATCCAGGTAAGAGAATCGCGTTGATGTCTCTTGTCCCCGTGTTTGTCCGTTTTTTGCCCGTGTTGGAGTTACCTCACTACGTTGGACGGACCTCATTTACTAC
BamHI

123 ACACAAGGCAATTGACCCACGCATGTATCTATCTCATTTTCTTACACCTTCTATTACCTTCTGCTCTCTCTGATTTGGAAAAAGCTGAAAAAAAAGGTTGAAACCAGTTCCCTGAAATTA
TGTGTTCCGTTAACTGGGTGCGTACATAGATAGAGTAAAAGAATGTGGAAGATAATGGAAGACGAGAGAGACTAAACCTTTTTCGACTTTTTTTTTCCAACTTTGGTCAAGGGACTTTAAT

243 TTCCCCTACTTGACTAATAAGTATATAAAGACGGTAGGTATTGATTGTAATTCTGTAAATCTATTTCTTAAACTTCTTAAATTCTACTTTTATAGTTAGTCTTTTTTTTTAGTTTTAAAAC
AAGGGGATGAACTGATTATTCATATATTTCTGCCATCCATAACTAACATTAAGACATTTAGATAAAGAATTTGAAGAATTTAAGATGAAAATATCAATCAGAAAAAAAATCAAATTTTG

                                                MetArgPheProSerIlePheThrAlaValLeuPheAlaAlaSerSerAlaLeuAlaAlaProValAsnThrThrThr
363 ACCAAGAACTTAGTTTCGAATAAACACACATAAACAAACACCATGAGATTTCCTTCAATTTTTACTGCAGTTTTATTCGCAGCATCCTCCGCATTAGCTGCTCCAGTCAACACTACAACA
TGGTTCTTGAATCAAAGCTTATTTGTGTGTATTTGTTTGTGGTACTCTAAAGGAAGTTAAAAATGACGTCAAAATAAGCGTCGTAGGAGGCGTAATCGACGAGGTCAGTTGTGATGTTGT
                                                                                     PstI

        GluAspGluThrAlaGlnIleProAlaGluAlaValIleGlyTyrLeuAspLeuGluGlyAspPheAspValAlaValLeuProPheSerAsnSerThrAsnAsnGlyLeuLeuPheIle
483 GAAGATGAAACGGCACAAATTCCGGCTGAAGCTGTCATCGGTTACTTAGATTTAGAAGGGGATTTCGATGTTGCTGTTTTGCCATTTTCCAACAGCACAAATAACGGGTTATTGTTTATA
CTTCTACTTTGCCGTGTTTAAGGCCGACTTCGACAGTAGCCAATGAATCTAAATCTTCCCCTAAAGCTACAACGACAAAACGGTAAAAGGTTGTCGTGTTTATTGCCCAATAACAAATAT

        AsnThrThrIleAlaSerIleAlaAlaLysGluGluGlyValSerLeuAspLysArgSerSerIleIleLeuGluSerGlyAsnValAsnAspTyrGluValValTyrProArgLysVal
603 AATACTACTATTGCCAGCATTGCTGCTAAAGAAGAAGGGGTATCTCTAGATAAAAGGAGCTCTATAATCCTGGAATCTGGGAACGTTAATGATTATGAAGTAGTGTATCCACGAAAAGTC
TTATGATGATAACGGTCGTAACGACGATTTCTTCTTCCCCATAGAGATCTATTTTCCTCGAGATATTAGGACCTTAGACCCTTGCAATTACTAATACTTCATCACATAGGTGCTTTTCAG
                                       XbaI            SacI

        ThrProValProArgGlyAlaValGlnProLysTyrGluAspAlaMetGlnTyrGluPheLysValAsnGlyGluProValValLeuHisLeuGluLysAsnLysGlyLeuPheSerGlu
723 ACTCCAGTGCCCAGAGGAGCAGTTCAGCCAAAGTATGAAGATGCCATGCAATATGAATTTAAAGTGAATGGAGAGCCAGTGGTCCTTCACCTGGAAAAAAATAAAGGACTTTTTTCAGAA
TGAGGTCACGGGTCTCCTCGTCAAGTCGGTTTCATACTTCTACGGTACGTTATACTTAAATTTCACTTACCTCTCGGTCACCAGGAAGTGGACCTTTTTTTATTTCCTGAAAAAAGTCTT

EP 0 323 722 A1

# FIG. 10-2

AspTyrSerGluThrHisTyrSerProAspGlyArgGluIleThrThrTyrProLeuValGluAspHisCysTyrTyrHisGlyArgIleGluAsnAspAlaAspSerThrAlaSerIle
GATTACAGCGAGACTCATTATTCCCCTGATGGCAGAGAAATTACAACATACCCCCTGGTTGAGGATCACTGCTATTATCATGGACGCATCGAGAATGATGCTGACTCAACTGCAAGCATC
CTAATGTCGCTCTGAGTAATAAGGGGACTACCGTCTCTTTAATGTTGTATGGGGGACCAACTCCTAGTGACGATAATAGTACCTGCGTAGCTCTTACTACGACTGAGTTGACGTTCGTAG

SerAlaCysAsnGlyLeuLysGlyHisPheLysLeuGlnGlyGluMetTyrLeuIleGluProLeuGluLeuSerAspSerGluAlaHisAlaValTyrLysTyrGluAsnValGluLys
AGTGCATGCAACGGTTTGAAAGGACATTTCAAGCTTCAAGGGGAGATGTACCTTATTGAACCGTTGGAGCTTTCCGACAGTGAAGCCCATGCAGTCTACAAATATGAAAATGTAGAAAAA
TCACGTACGTTGCCAAACTTTCCTGTAAAG**TTCGAAG**TTCCCCTCTACATGGAATAACTTGGCAACCTCGAAAGGCTGTCACTTCGGGTACGTCAGATGTTTATACTTTTACATCTTTTT
                            **HinDIII**

GluAspGluAlaProLysMetCysGlyValThrGlnAsnTrpGluSerTyrGluProIleLysLysAlaPheGlnLeuAsnLeuThrLysArgGlnGlnArgPheProGlnArgTyrVal
GAGGATGAGGCCCCCAAAATGTGTGGGGTAACCCAGAATTGGGAATCATATGAGCCCATCAAAAAGGCCTTTCAGTTAAATCTTACTAAAAGACAACAAAGATTCCCCCAAAGATACGTT
CTCCTACTCCGGGGGGTTTTACACACC**CCATTGGG**TCTTAACCCTTAGTATACTCGGGTAGTTTTTCCGGAAAGTCAATTTAGAATGATTTTCTGTTGTTTCTAAGGGGGTTTCTATGCAA
                          **BstEII**

GlnLeuValIleValAlaAspHisArgMetAsnThrLysTyrAsnGlyAspSerAspLysIleArgGlnTrpValHisGlnIleValAsnThrIleAsnGluIleTyrArgProLeuAsn
CAGCTGGTCATAGTTGCGGATCACAGAATGAATACGAAATACAATGGTGATTCAGATAAGATAAGACAATGGGTTCACCAGATTGTCAACACTATAAATGAGATTTACAGACCTTTGAAT
**GTCGAC**CAGTATCAACGCCTAGTGTCTTACTTATGCTTTATGTTACCACTAAGTCTATTCTATTCTGTTACCCAAGTGGTCTAACAGTTGTGATATTTACTCTAAATGTCTGGAAACTTA
**PvuII**

IleGlnPheThrLeuValGlyLeuGluIleTrpSerAsnGlnAspLeuIleThrValThrSerValSerHisAspThrLeuAlaSerPheGlyAsnTrpArgGluThrAspLeuLeuArg
ATTCAATTCACACTGGTTGGCCTAGAAATTTGGTCCAACCAAGATTTGATTACCGTGACATCAGTATCACATGATACTTTGGCCTCATTTGGAAACTGGAGAGAGACAGACTTGCTAAGG
TAAGTTAAGTGTGACCAACCGGATCTTTAAACCAGGTTGGTTCTAAACTAATGGCACTGTAGTCATAGTGTACTATGAAACCGGAGTAAACCTTTGACCTCTCTCTGTCTGAACGATTCC

ArgGlnArgHisAspAsnAlaGlnLeuLeuThrAlaIleAspPheAspGlyAspThrValGlyLeuAlaTyrValGlyGlyMetCysGlnLeuLysHisSerThrGlyValIleGlnAsp
CGCCAAAGACATGATAATGCCCAGTTACTCACGGCCATTGACTTTGATGGAGACACTGTAGGATTGGCTTATGTGGGCGGTATGTGCCAACTGAAGCACTCCACTGGAGTTATCCAGGAT
GCGGTTTCTGTACTATTACGGGTCAATGAGTGCCGGTAACTGAAACTACCTCTGTGACATCCTAACCGAATACACCCGCCATACACGGTTGACTTCGTGAGGTGACCTCAATAGGTCCTA

EP 0 323 722 A1

EP 0 323 722 A1

```
          HisSerAlaIleAsnLeuLeuValAlaLeuThrMetAlaHisGluLeuGlyHisAsnLeuGlyMetAsnHisAspGlyAsnGlnCysHisCysGlyAlaAsnSerCysValMetAlaAla
1563      CATAGTGCAATAAATCTTTTGGTTGCACTTACAATGGCCCATGAGCTGGGTCATAATCTGGGCATGAATCATGATGGAAATCAGTGTCATTGCGGTGCTAACTCGTGCGTCATGGCTGCC
          GTATCACGTTATTTAGAAAACCAACGTGAATGTTACCGGGTACTCGACCCAGTATTAGACCCGTACTTAGTACTACCTTTAGTCACAGTAACGCCACGATTGAGCACGCAGTACCGACGG

          MetLeuSerAspGlnProSerLysLeuPheSerAspCysSerLysLysAspTyrGlnThrPheLeuThrValAsnAsnProGlnCysIleLeuAsnLysProAM OC
1683      ATGTTAAGTGATCAACCCTCCAAACTATTCAGCGATTGTAGTAAGAAAGACTATCAGACGTTTCTTACGGTTAATAACCCACAATGCATTCTCAATAAACCCTAGTAAGTCGACTTTGTT
          TACAATTCACTAGTTGGGAGGTTTGATAAGTCGCTAACATCATTCTTTCTGATAGTCTGCAAAGAATGCCAATTATTGGGTGTTACGTAAGAGTTATTTGGGATCATTCAGCTGAAACAA
                                                                                              NsiI                    SalI

1803      CCCACTGTACTTTTAGCTCGTACAAAATACAATATACTTTTCATTTCTCCGTAAACAACATGTTTTCCCATGTAATATCCTTTTCTATTTTTCGTTCCGTTACCAACTTTACACATACTT
          GGGTGACATGAAAATCGAGCATGTTTTATGTTATATGAAAAGTAAAGAGGCATTTGTTGTACAAAAGGGTACATTATAGGAAAAGATAAAAAGCAAGGCAATGGTTGAAATGTGTATGAA

1923      TATATAGCTATTCACTTCTATACACTAAAAAAACTAAGACAATTTTAATTTTGCTGCCTGCCATATTTCAATTTGTTATAAATTCCTATAATTTATCCTATTAGTAGCTAAAAAAAGATGA
          ATATATCGATAAGTGAAGATATGTGATTTTTTGATTCTGTTAAAATTAAAACGACGGACGGTATAAAGTTAAACAATATTTAAGGATATTAAATAGGATAATCATCGATTTTTTTCTACT

2043      ATGTGAATCGAATCCTAAGAGAATTCGGATCC
          TACACTTAGCTTAGGATTCTCTTAAGCCTAGG
                             EcoRI  BamHI
```

# FIG. 10-3

pKS314

# FIG. II-I

1  GGATCCCCAGCTTAGTTCATAGGTCCATTCTCTTAGCGCAACTACAGAGAACAGGGGCACAAACAGGCAAAAAACGGGCACAACCTCAATGGAGTGATGCAACCTGCCTGGAGTAAATGATG
   CCTAGGGGTCGAATCAAGTATCCAGGTAAGAGAATCGCGTTGATGTCTCTTGTCCCCGTGTTTGTCCGTTTTTTGCCCGTGTTGGAGTTACCTCACTACGTTGGACGGACCTCATTTACTAC
   BamHI

123  ACACAAGGCAATTGACCCACGCATGTATCTATCTCATTTTCTTACACCTTCTATTACCTTCTGCTCTCTCTGATTTGGAAAAAGCTGAAAAAAAAGGTTGAAACCAGTTCCCTGAAATTA
    TGTGTTCCGTTAACTGGGTGCGTACATAGATAGAGTAAAAGAATGTGGAAGATAATGGAAGACGAGAGAGACTAAACCTTTTTCGACTTTTTTTTCCAACTTTGGTCAAGGGACTTTAAT

243  TTCCCCTACTTGACTAATAAGTATATAAAGACGGTAGGTATTGATTGTAATTCTGTAAATCTATTTCTTAAACTTCTTAAATTCTACTTTTATAGTTAGTCTTTTTTTTTAGTTTTAAAAC
    AAGGGGATGAACTGATTATTCATATATTTCTGCCATCCATAACTAACATTAAGACATTTAGATAAAGAATTTGAAGAATTTAAGATGAAAATATCAATCAGAAAAAAAATCAAAATTTTG

                                   MetArgPheProSerIlePheThrAlaValLeuPheAlaAlaSerSerAlaLeuAlaAlaProValAsnThrThrThr
363  ACCAAGAACTTAGTTTCGAATAAACACACATAAACAAACACCATGAGATTTCCTTCAATTTTTACTGCAGTTTTATTCGCAGCATCCTCCGCATTAGCTGCTCCAGTCAACACTACAACA
    TGGTTCTTGAATCAAAGCTTATTTGTGTGTATTTGTTTGTGGTACTCTAAAGGAAGTTAAAAATGACGTCAAAATAAGCGTCGTAGGAGGCGTAATCGACGAGGTCAGTTGTGATGTTGT
                                                               PstI

    GluAspGluThrAlaGlnIleProAlaGluAlaValIleGlyTyrLeuAspLeuGluGlyAspPheAspValAlaValLeuProPheSerAsnSerThrAsnAsnGlyLeuLeuPheIle
483  GAAGATGAAACGGCACAAATTCCGGCTGAAGCTGTCATCGGTTACTTAGATTTAGAAGGGGATTTCGATGTTGCTGTTTTGCCATTTTCCAACAGCACAAATAACGGGTTATTGTTTATA
    CTTCTACTTTGCCGTGTTTAAGGCCGACTTCGACAGTAGCCAATGAATCTAAATCTTCCCCTAAAGCTACAACGACAAAACGGTAAAAGGTTGTCGTGTTTATTGCCCAATAACAAATAT

    AsnThrThrIleAlaSerIleAlaAlaLysGluGluGlyValSerLeuAspSerSerIleIleLeuGluSerGlyAsnValAsnAspTyrGluValValTyrProArgLysValThrPro
603  AATACTACTATTGCCAGCATTGCTGCTAAAGAAGAAGGGGTATCTCTAGATAGCTCTATAATCCTGGAATCTGGGAACGTTAATGATTATGAAGTAGTGTATCCACGAAAAGTCACTCCA
    TTATGATGATAACGGTCGTAACGACGATTTCTTCTTCCCCATAGAGATCTATCGAGATATTAGGACCTTAGACCCTTGCAATTACTAATACTTCATCACATAGGTGCTTTTCAGTGAGGT
                                                     XbaI

    ValProArgGlyAlaValGlnProLysTyrGluAspAlaMetGlnTyrGluPheLysValAsnGlyGluProValValLeuHisLeuGluLysAsnLysGlyLeuPheSerGluAspTyr
723  GTGCCCAGAGGAGCAGTTCAGCCAAAGTATGAAGATGCCATGCAATATGAATTTAAAGTGAATGGAGAGCCAGTGGTCCTTCACCTGGAAAAAAATAAAGGACTTTTTTTCAGAAGATTAC
    CACGGGTCTCCTCGTCAAGTCGGTTTCATACTTCTACGGTACGTTATACTTAAATTTCACTTACCTCTCGGTCACCAGGAAGTGGACCTTTTTTTTATTTCCTGAAAAAAGTCTTCTAATG

# FIG. II-2

SerGluThrHisTyrSerProAspGlyArgGluIleThrThrTyrProLeuValGluAspHisCysTyrTyrHisGlyArgIleGluAsnAspAlaAspSerThrAlaSerIleSerAla
AGCGAGACTCATTATTCCCCTGATGGCAGAGAAATTACAACATACCCCCTGGTTGAGGATCACTGCTATTATCATGGACGCATCGAGAATGATGCTGACTCAACTGCAAGCATCAGTGCA
TCGCTCTGAGTAATAAGGGGACTACCGTCTCTCTTTAATGTTGTATGGGGGACCAACTCCTAGTGACGATAATAGTACCTGCGTAGCTCTTACTACGACTGAGTTGACGTTCGTAGTCACGT

CysAsnGlyLeuLysGlyHisPheLysLeuGlnGlyGluMetTyrLeuIleGluProLeuGluLeuSerAspSerGluAlaHisAlaValTyrLysTyrGluAsnValGluLysGluAsp
TGCAACGGTTTGAAAGGACATTTCAAGCTTCAAGGGGAGATGTACCTTATTGAACCGTTGGAGCTTTCCGACAGTGAAGCCCATGCAGTCTACAAATATGAAAATGTAGAAAAAGAGGAT
ACGTTGCCAAACTTTCCTGTAAAGTTCGAAGTTCCCCTCTACATGGAATAACTTGGCAACCTCGAAAGGCTGTCACTTCGGGTACGTCAGATGTTTATACTTTTACATCTTTTTCTCCTA
                               HinDIII

GluAlaProLysMetCysGlyValThrGlnAsnTrpGluSerTyrGluProIleLysLysAlaPheGlnLeuAsnLeuThrLysArgGlnGlnArgPheProGlnArgTyrValGlnLeu
GAGGCCCCCAAAATGTGTGGGGTAACCCAGAATTGGGAATCATATGAGCCCATCAAAAAGGCCTTTCAGTTAAATCTTACTAAAAGACAACAAAGATTCCCCCAAAGATACGTTCAGCTG
CTCCGGGGGGTTTTACACACCCCATTGGGTCTTAACCCTTAGTATACTCGGGTAGTTTTTCCGGAAAGTCAATTTAGAATGATTTTCTGTTGTTTCTAAGGGGGTTTCTATGCAAGTCGAC
                         BstEII                                                                                        PvuII

ValIleValAlaAspHisArgMetAsnThrLysTyrAsnGlyAspSerAspLysIleArgGlnTrpValHisGlnIleValAsnThrIleAsnGluIleTyrArgProLeuAsnIleGln
GTCATAGTTGCGGATCACAGAATGAATACGAAATACAATGGTGATTCAGATAAGATAAGACAATGGGTTCACCAGATTGTCAACACTATAAATGAGATTTACAGACCTTTGAATATTCAA
CAGTATCAACGCCTAGTGTCTTACTTATGCTTTATGTTACCACTAAGTCTATTCTATTCTGTTACCCAAGTGGTCTAACAGTTGTGATATTTACTCTAAATGTCTGGAAACTTATAAGTT

PheThrLeuValGlyLeuGluIleTrpSerAsnGlnAspLeuIleThrValThrSerValSerHisAspThrLeuAlaSerPheGlyAsnTrpArgGluThrAspLeuLeuArgArgGln
TTCACACTGGTTGGCCTAGAAATTTGGTCCAACCAAGATTTGATTACCGTGACATCAGTATCACATGATACTTTGGCCTCATTTGGAAACTGGAGAGAGACAGACTTGCTAAGGCGCCAA
AAGTGTGACCAACCGGATCTTTAAACCAGGTTGGTTCTAAACTAATGGCACTGTAGTCATAGTGTACTATGAAACCGGAGTAAACCTTTGACCTCTCTCTGTCTGAACGATTCCGCGGTT

ArgHisAspAsnAlaGlnLeuLeuThrAlaIleAspPheAspGlyAspThrValGlyLeuAlaTyrValGlyGlyMetCysGlnLeuLysHisSerThrGlyValIleGlnAspHisSer
AGACATGATAATGCCCAGTTACTCACGGCCATTGACTTTGATGGAGACACTGTAGGATTGGCTTATGTGGGCGGTATGTGCCAACTGAAGCACTCCACTGGAGTTATCCAGGATCATAGT
TCTGTACTATTACGGGTCAATGAGTGCCGGTAACTGAAACTACCTCTGTGACATCCTAACCGAATACACCCGCCATACACGGTTGACTTCGTGAGGTGACCTCAATAGGTCCTAGTATCA

EP 0 323 722 A1

EP 0 323 722 A1

```
        AlaIleAsnLeuLeuValAlaLeuThrMetAlaHisGluLeuGlyHisAsnLeuGlyMetAsnHisAspGlyAsnGlnCysHisCysGlyAlaAsnSerCysValMetAlaAlaMetLeu
1563    GCAATAAATCTTTTGGTTGCACTTACAATGGCCCATGAGCTGGGTCATAATCTGGGCATGAATCATGATGGAAATCAGTGTCATTGCGGTGCTAACTCGTGCGTCATGGCTGCCATGTTA
        CGTTATTTAGAAAACCAACGTGAATGTTACCGGGTACTCGACCCAGTATTAGACCCGTACTTAGTACTACCTTTAGTCACAGTAACGCCACGATTGAGCACGCAGTACCGACGGTACAAT

        SerAspGlnProSerLysLeuPheSerAspCysSerLysLysAspTyrGlnThrPheLeuThrValAsnAsnProGlnCysIleLeuAsnLysProAM OC
1683    AGTGATCAACCCTCCAAACTATTCAGCGATTGTAGTAAGAAAGACTATCAGACGTTTCTTACGGTTAATAACCCACAATGCATTCTCAATAAACCCTAGTAAGTCGACTTTGTTCCCACT
        TCACTAGTTGGGAGGTTTGATAAGTCGCTAACATCATTCTTTCTGATAGTCTGCAAAGAATGCCAATTATTGGGTGTTACGTAAGAGTTATTTGGGATCATTCAGCTGAAACAAGGGTGA
                                                                                   NsiI                         SalI

1803    GTACTTTTAGCTCGTACAAAATACAATATACTTTTCATTTCTCCGTAAACAACATGTTTTCCCATGTAATATCCTTTTCTATTTTTCGTTCCGTTACCAACTTTACACATACTTTATATA
        CATGAAAATCGAGCATGTTTTATGTTATATGAAAAGTAAAGAGGCATTTGTTGTACAAAAGGGTACATTATAGGAAAAGATAAAAAGCAAGGCAATGGTTGAAATGTGTATGAAATATAT

1923    GCTATTCACTTCTATACACTAAAAAAACTAAGACAATTTTAATTTTGCTGCCTGCCATATTTCAATTTGTTATAAATTCCTATAATTTATCCTATTAGTAGCTAAAAAAAGATGAATGTGA
        CGATAAGTGAAGATATGTGATTTTTTGATTCTGTTAAAATTAAAACGACGGACGGTATAAAGTTAAACAATATTTAAGGATATTAAATAGGATAATCATCGATTTTTTTCTACTTACACT

2043    ATCGAATCCTAAGAGAATTCGGATCC
        TAGCTTAGGATTCTCTTAAGCCTAGG
                 EcoRI  BamHI
```

# FIG. 11-3

pKS317

European Patent Office

**PARTIAL EUROPEAN SEARCH REPORT**
which under Rule 45 of the European Patent Convention shall be considered, for the purposes of subsequent proceedings, as the European search report

Application number

EP 88311924.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁴) |
|---|---|---|---|
| P,A | <u>CHEMICAL ABSTRACTS</u><br><br>vol. 108, no. 13, 28th March 1988; abstract no. 108585g; NED. B. EGEN et al.: "Isolation by preparative isoelectric focusing of a direct acting fibrinolytic enzyme from the venom of Agkistrodon contortrix contortrix (southern copperhead)"; & TOXICON 1987, vol. 25, no. 11, pages 1189-1198<br><br>---- | 1-17 | C12N15/00<br>C12N9/64<br>A61K37/00 |
| A | <u>CHEMICAL ABSTRACTS</u><br>vol. 94, no. 19, 11th May 1981; abstract no. 152588b; HUBERT PIRKLE et al.: "The primary structure of crotalase, a thrombin-like venom enzyme, exhibits closer | 1-17 | |

TECHNICAL FIELDS SEARCHED (Int. Cl.⁴)

C12N15/00
C12N9/64
A61K37/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-18
Claims searched incompletely:
Claims not searched: 19
Reason for the limitation of the search: Article 52(4) EPC

Method for treatment of the human or animal body by surgery or therapy

| Place of search | Date of completion of the search | Examiner |
|---|---|---|
| BERLIN | 20.03.1989 | P. JULIA Y BALLBE |

| Category | Citation of document with indication, where appropriate, of relevant passages | Relevant to claim | |
|---|---|---|---|
| | **DOCUMENTS CONSIDERED TO BE RELEVANT** | | CLASSIFICATION OF THE APPLICATION (Int. Cl.) |
| | homology to kallikrein than to other serine proteases"; & BIOCHEM. BIOPHYS. RES. COMMUN., 1981, vol. 99, no. 2, pages 715-721 | | |
| D,A | US - A - 4610879 (TRANCIS S. MARKLAND et al.)  * whole document * | 1-18 | |
| A | EP - A - 0123544 (GENENTECH., INC.)  * whole document * | 5-14 | TECHNICAL FIELDS SEARCHED (Int. Cl.) |

EPO Form 1505.3  06.78